# EUROPEAN PATENT APPLICATION

(11) **EP 4 520 377 A2**
(43) Date of publication of application: **12.03.2025**
(21) Application number: 25153300.6
(22) Date of filing: 22.01.2021
(51) Int. Cl.: A61M 16/06

(54) **AIR-JET DRY POWDER INHALER FOR RAPID DELIVERY OF PHARMACEUTICAL AEROSOLS TO INFANTS**

(30) Priority: 22.01.2020 US 202062964208 P
(62) Divisional of application: 21744961.0
(71) Applicant: Virginia Commonwealth University, Richmond, VA 23298 (US)
(72) Inventor: Longest, Worth, Richmond, 23298 (US); Howe, Connor, Richmond, 23298 (US); Hindle, Michael, Richmond, 23298 (US); Farkas, Dale, Richmond, 23298 (US); Strickler, Sarah, Richmond, 23298 (US); Rani, Vijaya, Richmond, 23298 (US); Elachway, Ahmad, Richmond, 23298 (US)
(74) Representative: Kröncke, Rolf

(57) **Abstract**

Proposed devices operate on positive pressure with as little as 5-6 ml of air and can efficiently empty (emitted doses >80%) and deliver the aerosol to infant lungs (lung delivery efficiency of ~60% of the loaded dose). Significant features include internal flow structure of the air-jet DPI, automatic gas sources, infant-specific interfaces, small diameter nasopharyngeal tubes, sealed nasal prongs, 3D rod array preceding patient interface, nasal CPAP rapid aerosol delivery system, nasal CPAP streamlined interface, multidose storage and delivery unit, and pressure sensing near the infant airways (at the nasal cannula interface).

## Description

### STATEMENT OF GOVERNMENT INTEREST

This invention was made with government support under Grant Numbers R01HD087339 and R01HL139673 awarded by the National Institutes of Health. The US government has certain rights in the invention.

### FIELD OF THE INVENTION

The invention is generally related to dry powder inhalers (DPIs) and, in particular, to DPIs and related apparatuses configured to minimize depositional losses and provide exemplary delivery of aerosolized therapeutics to infants.

### BACKGROUND

The industry is shifting to dry powder medications. There is a need for high dose inhalers. Commercial dry powder inhalers typically require approximately 3 to 4 Liters (L) of air to form an aerosol and empty a device. The best current commercial DPI requires approximately 1600 ml of air for operation. Infants only inhale on the order of 10 ml (~6 ml/kg) of air with each breath. As a result, current DPIs cannot be used with infants.

### SUMMARY

According to an aspect of some embodiments, proposed devices operate on positive pressure with as little as 5-6 ml of air and can efficiently empty (emitted doses >80%) and deliver the aerosol to infant lungs (lung delivery efficiency of -60% of the loaded dose). Significant features that are specific to infants and differentiate the disclosed technology include: internal flow structure of the air-jet DPI, automatic gas sources, infant-specific interfaces, small diameter nasopharyngeal tubes, sealed nasal prongs, 3D rod array preceding patient interface, nasal CPAP rapid aerosol delivery system, nasal CPAP streamlined interface, multidose storage and delivery unit, and pressure sensing near the infant airways (at the nasal cannula interface).

An exemplary air jet dry powder inhaler (DPI) system for infants includes a gas source configured to deliver in a single actuation up to but not exceeding a full inhalation breath volume for an infant, a patient interface configured to form an airtight seal with one or both of an infant's nostrils, and a fixed position air jet DPI arranged inline between the gas source and the patient interface and configured to introduce an aerosol to gas from the gas source before the gas reaches the patient interface. The air jet DPI system is configured to have airtight communication with lungs of the infant when the patient interface is forming an airtight seal with one or both of the infant's nostrils and all other pulmonary orifices are closed. The air jet DPI system delivers both the aerosol and the full inhalation breath volume for an infant using positive-pressure gas. The full inhalation breath volume delivered may be a maximum of 100 ml or less. The full inhalation breath volume may be a maximum of 10 ml or less. The total air space volume (the dead space) of the air jet DPI system may be 5 ml or less. A total air space volume of the air jet DPI system may be 2 ml or less. The total air space volume may be determined from air jet inlet orifice (capillary) through to the end of the patient interface, so parts would include the aerosolization chamber, outlet capillary, and patient interface. The gas source is configured to deliver full inhalation breath volume for an infant to the patient through the patient interface in one second or less. The system may comprise a bend in flow path downstream of the air jet DPI of 10° to 45°. The system may further include a drying chamber and a one-way valve configured for admitting air from the environment into the air jet DPI system when the gas source volume is expanding.

An exemplary gas source may include one or more hand actuated syringes. The one or more hand actuated syringes may comprise one or more springs. Another exemplary gas source may be a compressed gas source. The gas source may be a bank of gas syringes compressible prior to actuation. The gas syringes may be individually actuatable.

An exemplary patient interface may have a gradually expanding interior over a length of 40-80 mm. The patient interface may include a nasopharyngeal tube.

An exemplary air jet DPI may comprise an elongate aerosolization chamber with a longitudinal axis and one or more inlets and one or more outlets all positioned at an upper longitudinal segment of the aerosolization chamber. The upper longitudinal segment may extend no more than 50% of a length (or no more than 25%) of the aerosolization chamber.

An exemplary patient interface may include a 3D rod array arranged such that an aerosol jet entering the patient interface must pass through the 3D rod array before exiting the patient interface. The 3D rod array comprises a plurality of rows of rods which extend between opposite walls of a lumen of the patient interface. The exemplary 3D rod array spans less than an entire cross-sectional distance length of the lumen between the at least one inlet and the one or more exit orifices in a direction perpendicular to a long axis of the rods of the 3D rod array. The 3D rod array is spaced 0 to 5 mm (or 1 to 2 mm) away from the at least one inlet orifice along a primary flow axis of the lumen.

An exemplary gas source for an air jet dry powder inhaler (DPI) usable with infants comprises a barrel sized to retain 100 ml or less of gas (the barrel having an exit orifice at one end), a rod, a piston and gasket sealing an end of the barrel opposite the exit orifice and moveable by the rod to change an internal volume of the barrel by driving gas into or out of the exit orifice, a handle piece configured such that the rod, piston, and gasket are moveable a maximum displacement to deliver a predetermined volume of air through the exit orifice using a single squeeze.

An exemplary multidose storage and delivery unit (MDU) for dosing dry powder into an air jet dry powder inhaler (DPI) comprises a main body sized to accommodate 100 mg or less of powder and configured to attach in an airtight manner to an aerosolization chamber of the air jet DPI and a release mechanism for releasing predetermined doses from the main body into the aerosolization chamber of the air jet DPI upon satisfaction of a predetermined condition. The main body may be divided into compartments, each compartment accommodating a separate dose of the powder. The release mechanism is a rotatable retaining disk, and wherein the predetermined condition is a rotation of the retaining disk. Alternatively the release mechanism is a mesh or plate containing one or more small openings sized to limit or prevent passage of powder solely under the force of gravity, and the predetermined condition is a change in air flow or pressure at the one or more small openings.

An exemplary aerosol delivery system for infants may comprise a tubing for transporting a continuous positive airway pressure (CPAP) air supply; nasal prongs sealable with an infant's nostrils; at least one access port along the tubing next to the nasal prongs, the at least one access port comprising a temporarily removable cover and a receiving part, and the at least one access port being sized to allow passage of an aerosol delivery prong into a lumen of the tubing. The receiving part is sized to form an airtight seal with an end of the aerosol delivery prong which puts the delivery prong in fluid communication with at least one of the nasal prongs and the infant's airways.

Another exemplary aerosol delivery system for infants may comprise a tubing for transporting a continuous positive airway pressure (CPAP) air supply and one or more nasal prongs sealable with an infant's nostrils, wherein the one or more nasal prongs are in fluid communication with a lumen of the tubing and a conduit connecting with a single access port independent of the tubing to which an aerosol delivery prong is connectable. The conduit between the access port and the one or more nasal prongs allows the one or more nasal prongs to remain open. The conduit between the access port and the one or more nasal prongs is configured to deliver aerosol to the one or more nasal prongs with no sudden changes in flow direction. The conduit has an S-curve shape. The conduit has a total air volume of 0.5 ml or less.

According to an aspect of some embodiments, CPAP nasal prongs contain partial flow passages and a closable access port leading to the nasal prongs. When the surfactant-aerosol delivery prongs are not inserted, gas flow passes through the partial flow passage opening and into the nasal cannulas as usual. When the surfactant-aerosol delivery prong or prongs are inserted through the closable access port, an airtight flow passage is created between the air-jet DPI and the infants nasal airways while the nasal-CPAP prongs remain in place but the CPAP flow is temporarily bypassed. The temporary passage is used to deliver both aerosol and ventilation breaths to the infant for the period of surfactant-aerosol delivery, which is typically 1 to 5 breaths. Once the surfactant-aerosol is delivered, the delivery ports are removed, the access port is closed and nasal CPAP administration is resumed. Similar interfaces may be used which enable delivery through a nasal mask interface.

### BRIEF DESCRIPTION OF THE DRAWINGS

Figure 1 is an exemplary infant air-jet dry powder inhaler (DPI) system.
Figure 2A is another exemplary infant air-jet DPI system.
Figure 2B is a partial cross-sectional view of the DPI system of Figure 2A.
Figures 3A-3D are respectively side, cross-sectional side, top, and front views of an exemplary rod-piston actuator air source.
Figures 4A-4C are respectively side, cross-sectional side, and top views of another exemplary rod-piston actuator air source.
Figure 5A is an exemplary air source assembly in an exhaust mode.
Figure 5B is an exemplary air source assembly in a co-flow air mode.
Figure 5C is co-flow air delivery with aerosol delivery in a patient interface.
Figure 5D is an exemplary air source assembly with a microcontroller.
Figure 5E is another exemplary air source.
Figure 5F is exemplary air source with a bank of air syringes compressible prior to actuation.
Figure 5G is an end view of a bank of air syringes with separate actuation buttons for each syringe.
Figures 6A-6F are alternative air-jet DPI chambers including various inlet and outlet configurations.
Figures 7A and 7B are respectively side and top views of a multidose storage and delivery device.
Figures 8A and 8B are two variants of multidose storage and delivery devices which dose masses of powder to an aerosolization chamber during inhalation actuation.
Figure 9 is a patient interface with nasal cannula bifurcation after a 3D rod array.
Figure 10A is a patient interface with a long gradually expanding nasal cannula.
Figure 10B is a patient interface with a flexible nasopharyngeal tube interface with a length of e.g. 4 cm.
Figure 11 is a patient interface with a long gradual reduction in nasal cannula cross section after a 3D rod array.
Figure 12 is a patient interface with a jet diffuser such as a mesh or porous plate.
Figure 13A is a conventional nasal continuous positive airway pressure (CPAP) setup.
Figures 13B and 13C show alternate configurations for a nasal CPAP rapid aerosol delivery system which connects an air-jet DPI with an existing nasal CPAP bilateral nasal prong interface.
Figure 14 is another exemplary nasal CPAP rapid aerosol delivery system.
Figure 15 is results from Example 1 showing average cumulative primary particle size distribution [n=3] of the powder based on dispersion with RODOS at a pressure of 4 bar and measured with laser diffraction as a function of aerodynamic diameter (converted based on a formulation density of 1.393 g/cm³). The resulting mean (SD) MMAD was 1.17 (0.00).
Figure 16 is, for Example 1, pressure profile graphs of 30 ml AAVs using the hand operated syringe through devices D2, D5, and D6, measured at the air-jet DPI outlet [n=6].
Figure 17A is mean (SD) values of MMAD vs. ED based on device actuation into the NGI and AAVs of 30 ml using room-condition air. Linear best-fit trend lines are established among devices with similar performance.
Figure 17B is mean (SD) values of MMAD vs. ED based on device actuation into the NGI and AAVs of 10 ml using room-condition air. Linear best-fit trend lines are established among devices with similar performance.
Figure 18 is Mean (SD) values of MMAD vs. ED based on device actuation into the NGI for the three best performing devices actuated with 30 ml (open circles) or 10 ml (filled circles) of room-condition air.
Figure 19A is experimentally determined mean (SD) drug deposition fraction (based on loaded dose) grouped by region.
Figure 19B is the same information as Figure 19A but grouped by device. Anterior nose, middle passage (MP), and throat deposition fractions shown in Figure 19A are combined as NT deposition fractions in Figure 19B.

### DETAILED DESCRIPTION

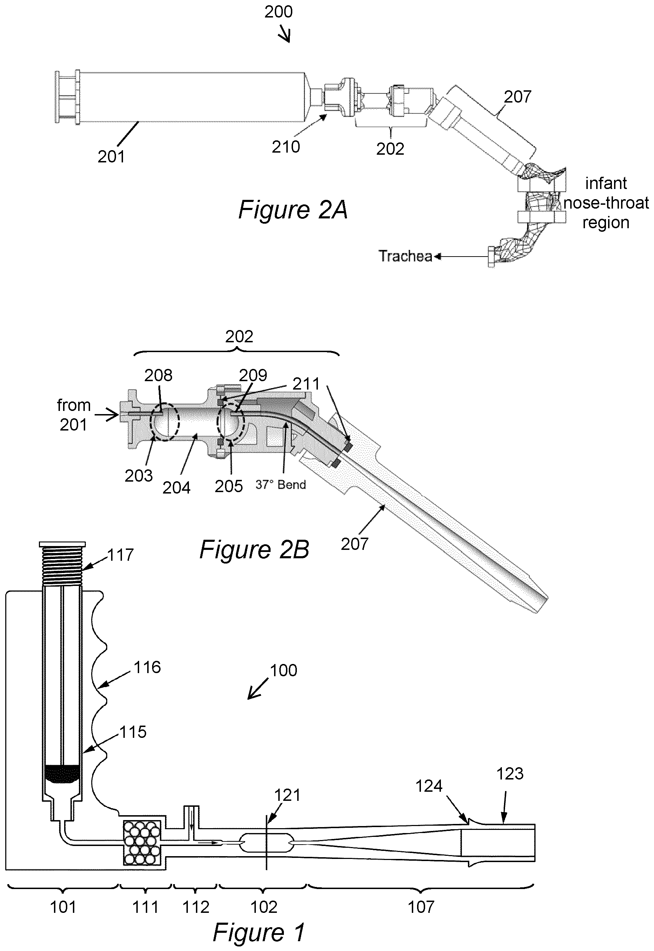

Figure 1 provides a comprehensive view of an exemplary infant air-jet dry powder inhaler (DPI) system 100. The system 100 may also be referred to as a rapid aerosol delivery (RAD) device.

The DPI system 100 comprises the following components: a gas source 101, a drying chamber 111, a one-way valve 112, an air-jet DPI 102, and a patient interface 107. To administer an aerosol, gas (e.g., air) moves through these components in the ordered listed, left-to-right in Figure 1. The one-way valve 112 is configured to allow air to enter the gas source 101 when the gas source 101 interior volume is expanding. The one-way valve 112 closes when the air source 101 is compressed. The drying chamber 111 is filled with, for example, a powder free desiccant material such as but not limited to silica gel beads. The drying chamber is sized to hold a desired volume of deliverable air such as 10, 20 or, 30 ml. The air in the drying chamber is pushed through the system 100 when the syringe 115 is compressed. The air in the syringe displaces the air in the drying chamber 111. When the syringe 115 is expanded again, air from the one-way valve 112 passes in the opposite direction through the drying chamber 111.

Operation of an exemplary DPI system 100 may be as follows. The air source is pre-filled and, if applicable, pre-compressed. Powder is loaded, e.g. as a capsule or a dose unit, in the DPI. The system 100 is then effectively sealed from the environment except for the outlet of the patient interface. The patient interface is then positioned with respect to the patient, e.g. prong is inserted in a nostril to achieve an airtight seal. If the patient interface has only a single nasal prong, the user of the system 100 closes the infant's remaining pulmonary openings, namely the other nostril and mouth. The closure is preferably just after exhalation nostril flair from the infant. Immediately after the system 100, in particular the gas source, is actuated. The system 100 delivers a volume of air corresponding with a full inhalation breath in addition to aerosol entrained by the gas. Then the user holds for 5-10 seconds. Next the infant's second nostril (or and/or mouth) is opened for a period of time allowing the infant several normal breaths. Then, the steps of closure of extra nostril (if applicable) and mouth, actuation, and hold are repeated. This cycle may be repeated 3-5 times to deliver the full dose desired. If the patient interface comprises two prongs, the interface may include an exhalation port to be used for ease of opening and closing the infant's free breathing between actuations.

The total air space volume (the dead space) of the air jet DPI system may be 5 ml or less (excluding the volume of the air source, which can be variable). A total air space volume of the air jet DPI system may be 2 ml or less. The total air space volume may be determined from air jet inlet orifice (capillary) through to the end of the patient interface, so pertinent parts would include the aerosolization chamber, outlet capillary, and patient interface.

While the listed components in Figure 1 are exemplary, some embodiments may use fewer components, add additional components, or interchange components. For instance, the drying chamber 111 may be omitted depending on the moisture/dryness requirements of the particular dry powder formulation being used or the ambient humidity. The one-way valve 112 may be combined with the gas source 101. Alternatively the one-way valve 112 may be omitted for some types of gas sources 101, various alternatives for which will be described below.

Figure 2A gives an overview of another exemplary infant air-jet DPI system 200. Figure 2B shows an enlarged cross-sectional view of some of system 200's components. At the center of the system 200 is the air-jet DPI 202, which comprises or consists of small diameter inlet flow passage 203, small diameter outlet flow passage 205, and an aerosolization chamber 204. The inlet and outlet may have sharpened hollow fixed capillaries to pierce a capsule containing a dry powder and provide an airflow passage through the aerosolization chamber. The aerosolization chamber 204 may be sized to match a standardized capsule size, such as a size zero capsule. The interior of the capsule then forms the inner flow pathway. Different from many other DPI embodiments, the capsule or aerosolization chamber does not move or vibrate during actuation. The device is actuated with a positive pressure gas source 201 which may be but is not limited to a hand-actuated syringe with a maximum volume of, for example, 10, 30, or 60 ml. The gas source 201 may be connected to the DPI 202 with an adapter 210 such as a Luer adapter.

Aerosol leaving the DPI 202 enters the patient interface 207 which may include but is not necessarily limited to a nasal cannula. An important aspect of the infant air-jet DPI system 200 is that the nasal interface 207 is configured to form an airtight seal with one or both of the patient infant's nostrils. Airtight communication with the infant's lungs is important such that with a single-prong interface the other nostril is preferably held closed. The infant's mouth is also required to be held closed, either manually or with a chin strap as commonly used during infant respiratory support with nasal interfaces. The cannula of the patient interface 207 may have a gradually expanding interior such that an inlet diameter of e.g. 0.9 mm grows gradually to an outlet diameter of e.g. 3 mm over a length of 40-80 mm. Exemplary patient interfaces 207 include single-prong nasal interfaces as well as dual-prong nasal interfaces. According to the embodiment shown by Figure 2B, the exterior distal end of the nostril interface has an expanded (conical) cross-section, which forms an airtight seal with the nostril when inserted approximately 5 mm into the infant's nostril.

Care is taken where separate parts are joined or where the system is intended to be opened by a user to ensure a generally airtight system when administering an aerosol. Figure 2B shows seals 211 where the DPI 202 is opened to insert a capsule into the chamber 204 and where the patient interface 207 connects with the air-jet DPI 202. Though not illustrated, a similar seal is provided at the separation point 121 of DPI 102 for loading a capsule in system 100 of Figure 1. Sealing with the patient's airways is assisted by, for example, use of flexible prong 123 on the patient interface 107 for insertion into the patient's nostrils and a wedge shaped expansion 124 on the outer side of the patient interface 107 for snugging against the sidewalls of the patient's nasal airways.

The exemplary infant air-jet DPI systems 100 and 200 deliver both the aerosol and a full inhalation breath to the infant in a short amount of time (typically < 1 sec for inhalation) and can be used to maintain a short breath hold. The use of positive pressure to deliver the aerosol and inhalation breath better expands the flexible upper airways and may enable deeper than tidal volume inhalation and improved lung penetration of the aerosol. As with manual ventilation with a bag and mask interface, this approach may also help to open closed or obstructed lung regions, further increasing the reach of the inhaled aerosol.

Delivered gas to form the aerosol and support infant respiration depends on infant weight with a typical range of 6-8 ml of gas per kg of infant body weight (i.e., 6-8 ml/kg). For a preterm infant weighting 1600 g, potential delivered gas volumes would range from 10 to 13 ml with a preferred value of approximately 10 ml. For a full-term infant weighting 3550 g, potential delivered gas volumes would range from 21 to 28 ml with a preferred values of approximately 21 ml. In both cases the targeted value was selected as the lower end of the range to prevent lung volutrauma; however, slightly higher values within the range may be preferred by the physician to better open the airways and prevent lung collapse and reopening (cyclic atelectasis), which is also a source of lung injury. Hence, it is important for the administering physician to have precise control over the amount of air delivered to the infant and that this air volume can be adjusted depending on the lung injury and specific case being treated. The lower end of delivered gas volume to extremely preterm infants would be 4-5 ml and the upper end to much older infants suffering acute respiratory distress may be as high as 100 to 200 ml.

For infant air-jet DPI systems such as those of Figures 1 and 2A/2B, several types of gas source (e.g., air source) devices may be employed. System 200 of Figure 2A illustrates one of the simplest gas sources, namely an air-filled syringe. System 100 of Figure 1 illustrates a slightly more complex gas source. The gas source 101 comprises a syringe 115 which may be, for example, 10, 30, or 60 ml volume. In addition, the gas source 101 comprises a handle grip 116 which, when connected with the other components of the system 100, permits a user (such as a medical professional or caretaker) to manipulate not only the syringe 115 but the entire system 100. The handle grip 116 therefore allows the user to comfortably and carefully position the DPI 102, for which orientation may be consequential, as well as the orientation of the patient interface 107 with respect to the patient. The gas source 101 further includes a spring return 117 which automatically and conveniently draws air back into the syringe 115 after the plunger is depressed and then released by the user. The spring return 117 has the advantage of permitting a user to operate the system 100 with a single hand, even when administering two or more inhalation volumes in succession. Holding the handle grip 116, a user can actuate the system 100 by pressing down the plunger with the thumb of the gripping hand, release the thumb to allow the syringe 115 to refill with air, then press down the plunger with the thumb of the gripping hand once again. This can be repeated as many times as desired or required for a particular aerosol administration (e.g., 1, 2, 3, 4, 5, 6, or more inhalation volumes).

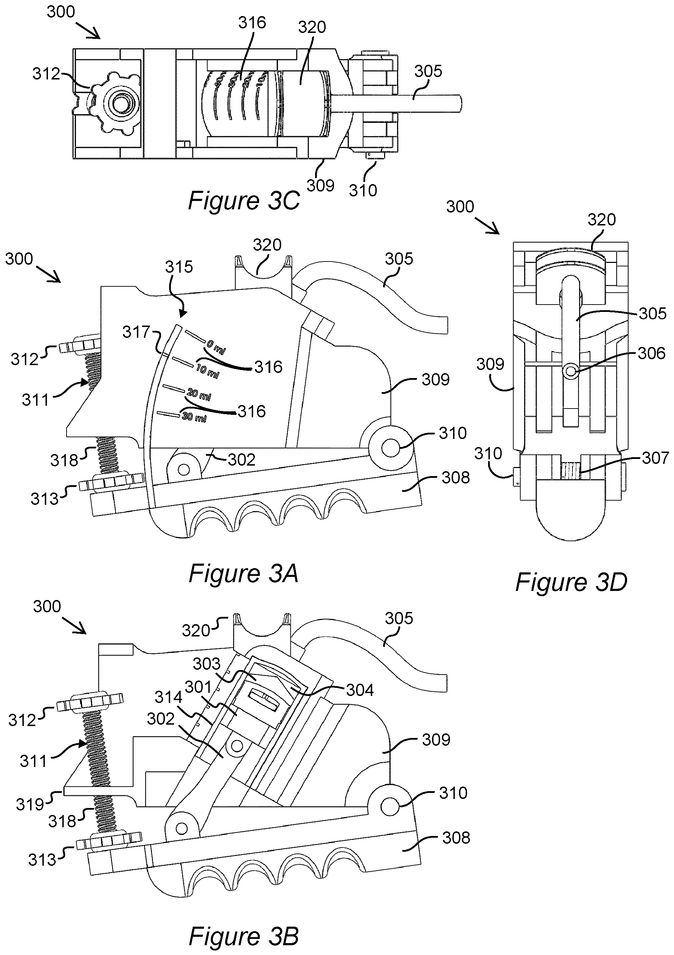

Figures 3A-3D show another exemplary air source 300. The air source 300 is a highly user friendly device premised on an air piston and rod configuration which is operable with a single hand. Figure 3B is a cross-sectional view that removes exterior housing to permit viewing of the piston 301, rod 302, and gasket 303. The air space 304 is enclosed by the gasket 303 and barrel 314 except for an outlet opposite gasket 303 which puts the air space 304 in fluid communication with the lumen 306 of outflow tubing 305. The lumen 306 connects downstream with, for example, a drying chamber 111, one-way valve 112, aerosolization chamber 102, and patient interface 107 as discussed above in connection with Figure 1. A spring 307, e.g. a torsion spring, maintains a return force on the bar of the hand grip 308 relative to the body 309 which houses the rod 302 and piston 301. The spring 307 is arranged to urge the hand grip 308 away from the body 309 about a hinge 310. The rod 302 is connected to the handle grip 308 at a position displaced along the handle grip 308 from the hinge 310. As a result, the spring 307 urges the rod 302 away from the start of the lumen 306 such that the space 304 is enlarged.

A volume limiter 311, which may include one or both of a maximum volume limiter knob 312 and a minimum volume limiter knob 313, is arranged to limit the displacement of the hand grip 308 from the body 309 rotationally about the axis of hinge 310. A shaft 318 of the volume limiter passes freely through a hole in a stop 319 of the body 309. The hole in the stop 319 is sized to allow passage of the shaft 318 but not of the limiter knobs 312 and 313. The volume limiter 311 effectively sets maximum and minimum displacements of the rod 302 within the barrel 314. The piston 301 is fixedly connected with the rod 302, and the gasket 303 is fixedly connected with the piston 301, such that all three components 301, 302, 303 move together and are limited together in maximum displacement within the barrel 314 by the volume limiter 311. The knobs 312 and 313 are slidable along the shaft of the volume limiter 311 via a rotational motion exerted by a user to vary the maximum and minimum volumes of air space 304. The barrel may be sized to retain up to 100 ml, or 100 ml or less, of gas to accommodate most infants. Infants are classified as 2 years old and under. For example, given a max infant weight of 15 kg at 2 years * 6 ml/kg = 90 ml delivery may be desired.

A volume position indicator 315 gives a user a clear readout of the current volume of air space 304 based on the current setting of the maximum volume limiter knob 312. The volume position indicator 315 comprises first indicia 316 which represent respective volume levels, e.g. 0 ml, 10 ml, 20 ml, and 30 ml. The first indicia have fixed positions with respect to the body 309 and barrel 314 housed by the body 309. A second indicium 317 has a fixed position with respect to the handle grip 308 and moves with respect to the first indica 316 whenever the handle grip 308 moves with respect to the body 309. Prior to the application of any external force by a user to handle grip 308, the spring 307 causes maximum displacement of the handle grip 308 with respect to the body 309 as limited by the volume limiter 311, in particular the maximum volume limiter 312. In this configuration, the second indicium 317 will come to rest next to a first indicium 316 which conveys the present volume of air space 304 that will be delivered by the air source 300 through lumen 306 if actuated.

A user actuates the air source 300 by moving the handle grip 308 toward the body 309. This may be achieved through a one-handed squeezing action in which the user has a thumb placed on thumb grip 320 and the remainder of his or her fingers placed on the handle grip 308. It should be noted that air source 300 is depicted in a partially actuated state, as though a user is midway through a full squeeze action. This is apparent from the fact that neither of the knobs 312 and 313 are in contact with the stop 319. In a fully expanded state of the air source 300 in which a user has not applied any rotational force exceeding that of the spring 307, the knob 312 is in contact with the stop 319. In a fully compressed state of the air source 300 in which a user has applied a rotational force exceeding that of the spring 307 and displaced the hand grip 308 a maximum distance toward the body 309, the knob 313 comes into contact with the stop 319. In embodiments that do not include a minimum volume limiter knob 313, the handle grip 308 itself may reach and contact the stop 319.

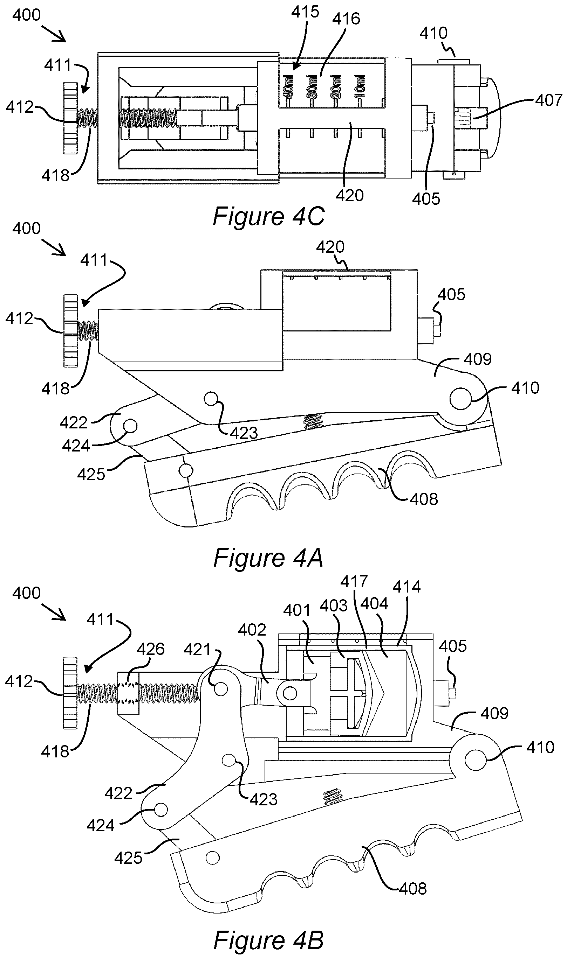

Figures 4A-4C show yet another exemplary air source 400. Like air source 300, air source 400 is based on a piston rod configuration. The cross-sectional view of Figure 4B shows the piston 401, rod 402, and gasket 403. The air space 404 is enclosed by the gasket 403 and barrel 414. The air space 404 has one outlet opposite the gasket 403 which puts the air space 404 in fluid communication with the lumen of outflow tubing 405. The lumen connects downstream with, for example, a drying chamber 111, one-way valve 112, aerosolization chamber 102, and patient interface 107 as discussed above in connection with Figure 1. A spring 407, e.g. a torsion spring, maintains a return force on the bar of the hand grip 408 relative to the body 409 which houses the rod 402 and piston 401. The spring 407 is arranged to urge the hand grip 408 away from the body 409 about a hinge 410. The rod 402 is connected to a first moving pivot 421 of a bell crank 422. A fixed pivot 423 of the bell crank 422 is positioned on the body 409. A second rod 425 connects the second moving pivot 424 of the bell crank 422 with a distal end of the handle grip 408 opposite the end of the handle grip where the hinge 410 is arranged. This collective configuration has the effect that spring 407 urges the rod 402 away from the start of the lumen of outlet tube 405 such that the space 404 is enlarged.

A volume limiter 411 comprises a maximum volume limiter knob 412 and a threaded shaft 418. The knob 412 is fixed to the end of the shaft 418. The shaft 418 passes through a threaded hole 426 of the body 409. Rotation of the knob 412 by a user displaces the first moving pivot 421 along the axis of the shaft 418 (left or right according to the orientation in Figure 4B) which adjust the maximum volume of air space 404. The piston 401 is fixedly connected with the rod 402, and the gasket 403 is fixedly connected with the piston 401, such that all three components 401, 402, 403 move together and are limited together in maximum displacement within the barrel 414 by the volume limiter 411. A volume position indicator 415 gives a user a clear readout of the current volume of air space 404 based on the current setting of the maximum volume limiter knob 412. The volume position indicator 415 comprises first indicia 316 which represent respective volume levels, e.g. 0 ml, 10 ml, 20 ml, 30 ml, and 40 ml. The first indicia have fixed positions with respect to the body 409 and barrel 414 housed by the body 409. A second indicium 417 moves with respect to the first indica 416 whenever the handle grip 08 moves with respect to the body 409. The second indicium 417 may be a physical element e.g. in this illustrative case a physical edge of the gasket 403 visible through a transparent wall of the barrel 414. Prior to the application of any external force by a user to handle grip 408, the spring 407 causes maximum displacement of the handle grip 408 with respect to the body 409 as limited by the volume limiter 411. In this configuration, the second indicium 417 will come to rest next to a first indicium 416 which conveys the present volume of air space 404 that will be delivered by the air source 400 through outlet tube 405 if actuated.

A user actuates the air source 400 by moving the handle grip 408 toward the body 409. This may be achieved through a one-handed squeezing action in which the user has a thumb placed on thumb rest bar 420 and the remainder of his or her fingers placed on the handle grip 408. A minimum volume limiter 413 may be included in some embodiments. The minimum volume limiter 413 has a variable length and extends between the handle grip 408 and the body 409. The minimum volume limiter 413 sets a minimum displacement between the handle grip 408 and body 409 which, in turn, sets the lower limit of volume for air space 404.

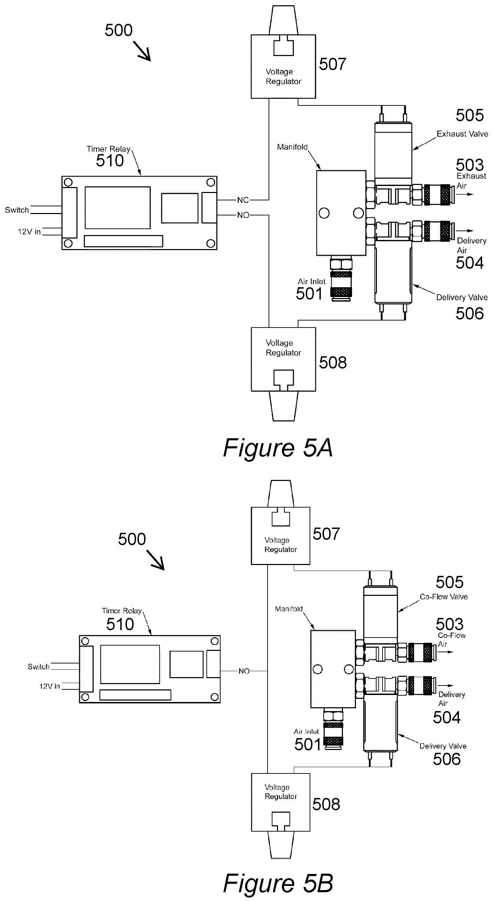

Figure 5A is an exemplary air source assembly 500. The air inlet 501 is configured to connect to a compressed gas source (e.g., approx. 50-100 psi) such as a wall air outlet or a compressed air tank (not shown). A regulator (not shown) may be arranged between the compressed gas source and the air inlet 501 to reduce the starting pressure to a safe level for the manifold 502 and subsequent components. The manifold 502 splits air from air inlet 501 into two outlets 503 and 504. Outlet 503 is an exhaust air outlet. Outlet 504 is a delivery air outlet. Each outlet 503 and 504 is regulated using individual flow regulation valves 505 and 506, respectively, which may be solenoids. The valves 505 and 506 are connected respectively to voltage regulators 507 and 508. The outlet 504 is used to deliver air through the DPI system (e.g., 100 of Figure 1) for aerosol delivery. The outlet 504 is connected to the normally open (NO) relay of a timer 510. The outlet 503 has two functions depending on the desired mode. In a first mode, air is released as exhaust air through outlet 503 to prevent a pressure buildup on the delivery valve 504 for air volumes of e.g. 300 ml and above used with children and adults. This mode may not be necessary for low volume / low flow delivery used with infants. The valve 505 is connected to the normally closed (NC) relay of the timer 510.

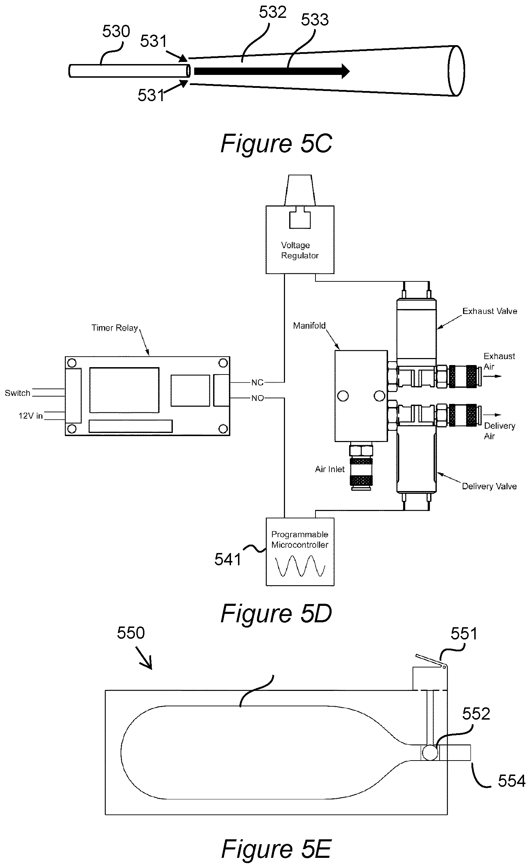

Figure 5B shows the air source assembly 500 configured in a second mode. In the second mode, co-flow air is actuated simultaneously from the outlet 503 as from outlet 504. In effect two pulses of air are produced simultaneously but independently of one another. Co-flow air that is actuated simultaneously with the delivery air to produce a second pulse of air that can be adjusted independently from the delivery air. Both valves 505 and 506 are connected to the normally on (NO) relay of the timer 510. Figure 5C provides a visual of the use of co-flow air. The co-flow air 531 is passed around (bypasses) the air-jet DPI and enters the patient interface at the patient interface inlet as a ring of aerosol free air 532 around a central aerosol stream 533 coming from the DPI capillary 530. This configuration serves the purpose of reducing patient interface depositional loss.

In either of the modes depicted by Figures 5A and 5B, the timer relay 510 is set to run air out of one or more of the outlets 504 and 503 depending on the desired pre-set delivery volume. Flow rates through the respective valves is set using the voltage regulators 507 and 508. A button for user activation may be provided. When the button, which is connected to the switch terminals on the timer as a momentary switch, is activated, the system delivers a single pulse of air through the valve or valves for the specified time.

In some of the above mentioned air sources, a user is required to physically impart energy on the air source to actuate the air source. The user's role may be substituted with one or more small motors which can be activated electronically by a controller such as a computer or microprocessor. In some embodiments pneumatic actuation or electromechanical actuation (e.g., a syringe pump) may be used. In for example pneumatic actuation, a pneumatic actuator replaces the hand and lever mechanism in Figure 4B and is used to move the air-tight piston head 403 with energy from a compressed gas source. Embodiments may also include a component or device that imparts fluctuations in pressure and/or flow delivered to the air-jet DPI, e.g. oscillation in input pressure or flow, as illustrated in Figure 5D by a programmable microcontroller 541. Whether manual or automatic, exemplary gas sources may operate with exemplary operation conditions such as delivery time of ~0.3 s; delivery volume of 10 ml; delivery flow rate of 1.2 to 5 LPM, e.g. ∼ 2 LPM; or delivery pressure received by the infant (measured at the infant interface) of 15 to 40 cm H₂O with a range of 20 to 25 cm H₂O to best prevent lung damage.

Figure 5E shows another air source alternative. Portable air source 550 uses compressed gas releasable in a small amount appropriate for aerosol delivery to infants via a trigger mechanism. A user actuatable trigger 551 connects to a valve 552 which controls the release of gas from compressed gas chamber 553 to air source outlet 554. The trigger switch 551 and/or valve 552 are configured or adjustable to release a predefined amount of gas appropriate for infants (e.g., a full inhalation volume for an infant). The gas in chamber 553 may be, for example, greater than or equal to 25 cm H₂O and have a volume sufficient to deliver at least 3 to 5 actuations (of say 10ml uncompressed gas each) without a significant drop (e.g., 30%) in internal pressure.

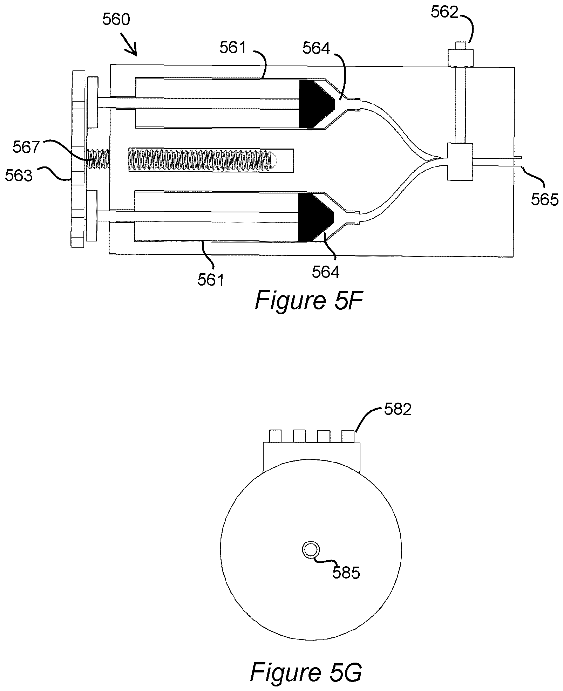

Figure 5F shows yet another air source. Air source 560 comprises a bank of two or more compressed syringes 561 with push button 562 actuation. The number of syringes 561 may vary among embodiments, e.g. 4, 6, 9, or some other number of syringes 561 may belong to a single bank sharing a single housing. A syringe compressor 563 is rotatable to advance a plate thereof toward or away from the syringes 561. The syringe compressor 563 resists translational movement that does not involve rotation of the plate by screw 567. A rotation in the e.g. clockwise direction compresses the syringes 561, or more specifically the enclosed air spaces 564. The compressed volumes of air in spaces 564 are then individually releasable for purposes of actuating a DPI connected downstream of outlet 565 using one of the button 562. An e.g. counterclockwise rotation of the compressor 563 or a quick release may be used to allow the syringes to be refilled with air (or another gas).

Figure 5G shows an end view of a bank of syringes, like the banks of Figures 5F, where there are four separatably actuatable buttons 582 which separately release predetermined volumes of compressed gas to a singular outlet 585.

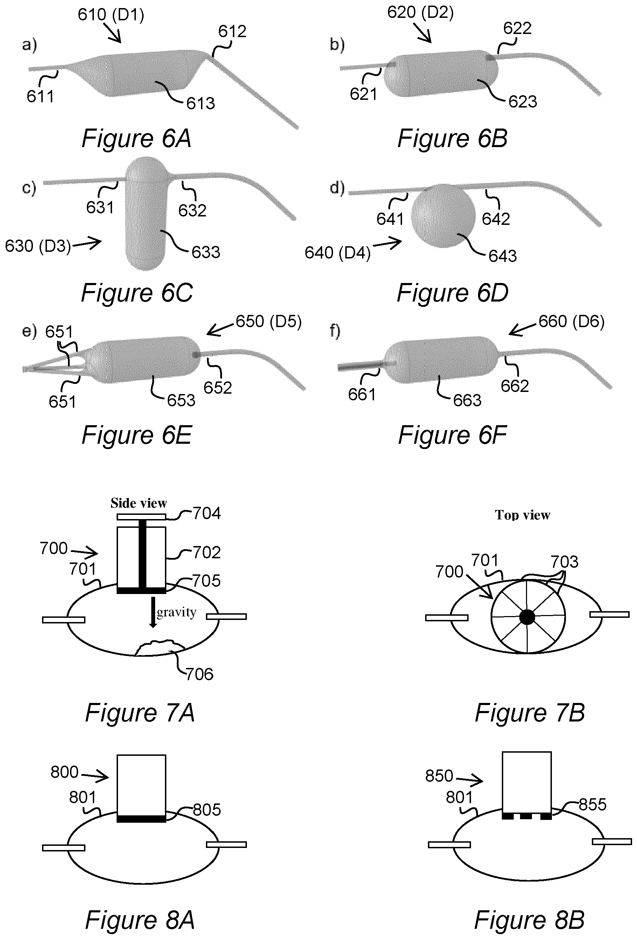

Figures 6A to 6F show alternative inlet air-jet configurations, where airflow would move left to right according to the illustrated orientations. Figure 6A is an air-jet DPI 610 with a horizontal cylindrical aerosolization chamber 613. Inlet 611 and outlet 612 are near a top side of the chamber 613. The cross-diameter of the chamber 613 gradually reduces at either end to the cross-diameter sizes of the inlet 611 and outlet 612.

Figure 6B is an air-jet DPI 620 with a horizontal cylindrical aerosolization chamber 623. Inlet 621 and outlet 622 are near a top side of the chamber 623. The inlet 621 and outlet 622 both project partially into a interior of the chamber 623.

Figure 6C is an air-jet DPI 630 with a vertical elongate (e.g. cylindrical) aerosolization chamber 633. Inlet 631 and outlet 632 are near a top side of the chamber 633. The air jet axis is perpendicular to the longitudinal axis of the aerosolization chamber and passes only through an upper longitudinal segment of the aerosolization chamber. The upper longitudinal segment extends no more than 50%, or 25%, of a length of the aerosolization chamber.

Figure 6D is an air-jet DPI 640 with a cylindrical aerosolization chamber 643. Inlet 641 and outlet 642 are near a top side of the chamber 643.

Figure 6E is an air-jet DPI 650 with a horizontal cylindrical aerosolization chamber 653. There are three inlets 651 arranged symmetrically about one longitudinal end of the chamber 653. The outlet 652 is positioned along the primary/center longitudinal axis of the chamber 653.

Figure 6F is an air-jet DPI 660 with a horizontal cylindrical aerosolization chamber 663. The inlet 661 and outlet 662 are both positioned at either of the longitudinal ends of the chamber 663 along the primary/center longitudinal axis of the chamber 663.

The outlet flow passages (right-hand-side of each of Figures 6A-6F) may be a single (as shown in all six figures) or double configuration leading to two nasal cannulas. The configurations of Figures 6A, 6B, 6E, and 6F implement a horizontal cylindrical chamber (HC). The configuration of Figure 6C comprises a vertical cylindrical chamber (VC). The configuration of Figure 6D comprises a spherical chamber (S). Inlet and outlet conditions for various exemplary air-jet DPI embodiments may include the options of rounded (R), flush (F), protruding within the aerosolization chamber (P), and multiple inlets (M). Details of the air-jet embodiments illustrated, referred to as D1-D6 (corresponding with Figures 6A-6F respectively), are provided in Table 1. Figure 2B illustrates the D2 embodiment including an outlet to form a 37° bend and attached to the infant nasal interface.

**Table 1: Air-jet dry powder inhaler (DPI) design parameters and configurations.**

| **DPI Device** | **Inlet** | **Chamber** | **Outlet** |
|---|---|---|---|
| D1 | R | HC | R |
| D2 | P | HC | P |
| D3 | F | VC | R |
| D4 | F | S | F |
| D5 | R/M* | HC | P |
| D6 | P-R/M** | HC | F |
| Chamber parameters include horizontal cylindrical (HC), vertical cylindrical (VC), or spherical (S). Inlet/Outlet parameters include rounded (R), flush (F), | | | |
| protruding (P), and/or multi (M). *Triple diverging inlet jets **Single protruding central jet, surrounded by triple parallel central jets | | | |

When the infant air-jet DPI is in use, the inlet flow passage is held in the horizontal position with respect to gravity and with the infant lying in the supine position. This orientation forms a bed of un-aerosolized powder on the floor of the aerosolization chamber prior to actuation. The complete internal flow passage of the air-jet DPI is required to curve through an angle of e.g. 10° to 45° for the nasal interface to correctly fit and seal with the infant's nostrils (see, e.g., exemplary bend of 37° in Figure 2B). Figures 6A-6F illustrate how different air-jet DPI configurations may achieve this change in flow direction using the outlet capillary of the infant air-jet DPI.

Briefly, exemplary air-jet DPIs behave as follows. A high velocity jet of air enters the aerosolization chamber, expands creating secondary velocities, and exits through the outlet orifice. The inlet and outlet orifices are typically aligned. The inlet air jet does not impinge on the initial bed of powder. Instead, secondary velocities initially form the aerosol. Typical operating conditions for an infant are 3 to 30 ml of actuation air for infants, but may be as low as 0.3 ml for animals, or higher than 30 ml for larger infants and children (but a unique challenge is good performance with low air volumes). Diameter of inlet flow passage may be 0.3 to 1 mm; preferred for some embodiments is 0.5 to 0.6 mm. Diameter of outlet flow passage: 0.5 to 1.2 mm, or 0.6 to 1.17 mm; preferred for some embodiments is 0.89 mm.

Figures 7A and 7B show a multidose storage and delivery unit (MDU) 700. An exemplary MDU 700 is attachable to a top of an aerosolization chamber 701 of an air-jet DPI via an airtight seal. For convenience of illustration a cylindrical horizontal aerosolization chamber is illustrated, but it will be appreciated that the MDU 700 is suitable for combination with any of the aerosolization chambers of Figures 6A-6F and alternatives thereto. The MDU 700 can be preloaded with one or more (e.g., a plurality) of doses of dry powder. Figure 7B shows how the multidose unit body 702 may be subdivided into a plurality of subchambers/compartments 703. Figure 7B shows eight compartments 703 of equal size, although the number of subchambers may be fewer or greater than this depending on the maximum dose per chamber desired (limited by each subchamber's volume). A dose release selector 704 allows a user to manually adjust which of subchambers open and which are closed. The dose release selector 704 may also be automated and actuated by a controller such as a computer or microprocessor.

Each compartment 703 is filled with a defined powder mass or deliberately left empty, depending on the amount of dosing desired for a particular patient. The powder mass may be equal among chambers (e.g., 10 mg), or it may vary, e.g., increase with each actuation. Rotation of the dose release selector 704 doses one or more masses of powder 706 to the aerosolization chamber 701 prior to inhaler actuation. Increasing the powder mass with each actuation may be beneficial if initial actuations have very limited air volumes to remain below safe delivery pressures due to non-compliant infant lungs associated with respiratory distress and surfactant dysfunction. As surfactant powder is delivered, the lungs become more compliant allowing for larger air volumes to be safely delivered, which can more efficiently aerosolize larger powder masses. The first compartment to be actuated may be deliberately left empty. This enables an administering clinician to ensure that good fluid communication is established with the patient's lungs prior to delivering the first dose of powder from a subsequent compartment of the MDU 700. For very small infants, only one or two compartments may be loaded delivering 10 -30 mg of powder in total. For full term or larger infants, 7 or more chambers may be loaded, delivering 70 mg or more of total powder.

A rotatable retaining disk 705 is positioned at a bottom of the MDU 700 with an opening or openable door sized to match one of the compartments 703. In the case of Figures 7A and 7B, in which the MDU volume is subdivided into eight compartments 703, the retaining disk 705 may have an opening of 1/8^{th} the cross-sectional area of the body 702 of the MDU 700. To load powder into the aerosolization chamber 701, the disk 705 is rotated 1/8 turn using the dose release selector 704, causing the contents of one chamber to fall into the aerosolization chamber 701. The device is then actuated with air to form the aerosol, clearing the aerosolization chamber of powder. Other retaining devices besides a rotating disk may be used in place of the exemplary disk 705 to contain the powder in the MDU 700 until such time as a user intends for the powder's release into the aerosolization chamber 701.

Figures 8A and 8B shows alternative MDUs 800 and 850 which does not have a rotating disk 705 as in MDU 700. The MDU 800 is covered on the bottom by a mesh 805, the MDU 850 by a plate 855 with openings. The MDUs 800 and 850 are loaded with a total intended delivery dose and capped until use. At the time of aerosol administration, the MDU is uncapped, inverted, and attached to the aerosolization chamber 801 of the air-jet DPI with an airtight seal (e.g., twist lock with o-ring). This class of MDUs employ barriers containing one or more small openings sized to limit or prevent passage of powder solely under the force of gravity. Thus, when the MDU 800 or 850 is installed or attached to the top of an aerosolization chamber 801, limited or ideally no powder at all falls from the MDU 800 into the aerosolization chamber 801. Instead of releasing powder prior to inhaler actuation, the MDU 800 release powder during inhaler actuation. It is therefore desirable that the interior of the MDUs 800 and 850 contained streamlined surfaces (e.g., few or no sharp corners) that minimize the ability for powder to be retained undesirably. The shape of the body of the MDU 800 or 850 may be a cylinder for example. Subdivision of the MDU body into compartments is not needed. With each device air actuation, the resulting air flow and pressure in aerosolization chamber 801 causes small air currents to pass through the small openings of the mesh 805. The small air currents entrain powder from within the MDU 800 or 850 and drive it into the aerosolization chamber 801 and onward to the patient. The size and number of the small openings in the mesh or plate may be calibrated to control the dosing for particular applications. For instance, the use of 2 or 3 small openings (~0.5 to 1 mm diameter) in fluid communication with the interior of the MDU 800 and the aerosolization chamber 801 may enable emptying of~30 mg of powder in 3 actuations. Ideally the number and size of openings or mesh size would enable the delivery of approximately 10 mg of powder per air actuation. Generally the number of small openings is at least two to generate internal flow patterns sufficient to remove powder remnants once the bulk of the powder in the MDU is delivered.

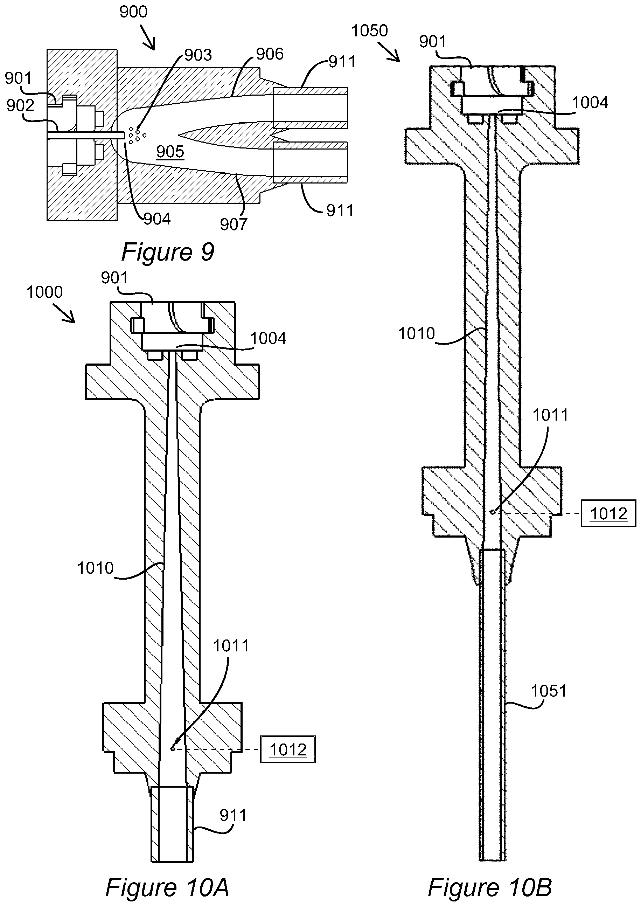

Figure 9 to Figure 12 show various exemplary patient interfaces. In the interest of brevity, a few features common to all four embodiments will be discussed first. Each of the patient interfaces 900, 1000, 1100, and 1200 comprises one or two flexible prongs 911. A flexible prong 911 may be a small diameter (e.g., approx. 3mm) short nasal prong or long nasal prong. Short nasal prongs avoid significant insertion. Long nasal prong bypasses the nasal valve and associated loss, but is not as long as a nasopharyngeal tube. A nasal prong 911 may be inserted the first 2-15 mm into the nasal passages, target 4-11mm. The length of the prongs 911 may be made to correspond with the target insertion distance or may be a little longer. Generally, the longer the inserted element, the more nasal dead space can be bypassed. Bypassing such dead space improves penetration of the infant lungs, especially when using a limited volume of delivery gas. For example, with flexible prongs 911 configured to bypass dead space in the nasal cavity, of a 10 ml of inhalation delivery, 2 ml may be wasted in the nasal airway dead space. The flexible nasal prongs 911 are sized and made of such material (e.g., silicone) to seal to one or both nostrils of the infant. More specifically, the outer diameter of the prongs 911 are sized to provide a seal with typical infant nostril size. The shape of the prongs 911 may be cylindrical with uniform outer diameter along the length of the prong or conical with a reducing outer diameter along the length of the prong in the direction of the patient. Exemplary inner diameters for fitting with infant nostrils include 1.4 to 5 mm with typical values of 3 mm for preterm and 4 mm for full term infants. Approximately 3 mm is generally better for preterm infants, whereas approximately 4 mm is generally better for full term infants.

Each of the patient interfaces 900, 1000, 1100, and 1200 further comprises a receiver port 901 configured to receive an upstream portion of the air-jet DPI system (e.g., refer back to systems 100 and 200 of Figures 1 and 2A/2B). For instance, the receiver port 901 may be configured to receive and attach in an airtight manner with the outlet end of the air-jet DPI, e.g., any of the outlets from the exemplary air-jet DPIs of figures 6A-6F. It should be noted that while the interfaces 900, 1000, 1100, and 1200 all show a single inlet orifice, multiple such interfaces may be combined to accommodate a plurality of inlets in the case the air-jet DPI has a plurality of outlets.

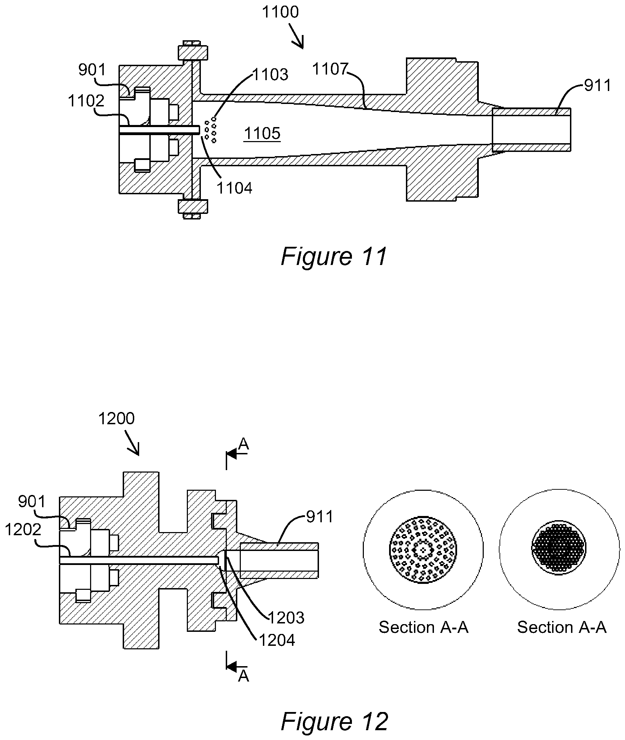

Referring now to Figures 9 and 11 specifically, in both patient interfaces 900 and 1100 a 3D rod array 903 or 1103 is arranged immediately after the inlet orifice 904 or 1104 to the main cannula 905 or 1105 of the patient interface. The inlet orifices 904 and 1104 are positioned within the respective cannulas 905 and 1105 past the distalmost end of the cannula. The 3D rod arrays 903 and 1103 comprise a plurality of parallel rods organized into a plurality of rows (distinguishing it from, for example, 2D grids or meshes). Suitable rods for some embodiments are disclosed by U.S. Patent No. 10,105,500 B2 which is incorporated herein by reference.

Generally, a 3D rod array 903 may be characterized by a plurality of rows each of which has a plurality of unidirectional rods disposed within a flow passage of an inhaler and spaced apart along a primary direction of air flow in the flow passage. A primary direction of air flow in the flow passage may be described as a longitudinal direction or z-direction of the flow passage. Successive unidirectional rows in a primary direction of air flow may or may not lie on the same line and are preferably staggered. This generally means that the rods of a first row in a first x-y plane of the flow passage and the rods of a second row in a second x-y plane of the flow passage are not in direct alignment with each other in the z-direction. The rows are preferably parallel to one other, and the rods are generally parallel to one another. In a preferred embodiment the rods in the second x-y plane are offset by 1-99% (most preferably 50%) from the rods in the first x-y plane such that air flowing (generally with increased velocity) between two rods of the first row in the first plane impacts on one or more rods (preferably the centers of the rods) of the second row in the second plane. In a preferred embodiment, all the rods of the plurality of rows of a 3D rod array are oriented in a same direction. Figure 9 provides a cross-sectional view of an exemplary embodiment of a 3D rod array wherein there are three rows of rods and each successive row is offset by 50% from the preceding row such that air flowing between two rods impacts on the center of a rod in a subsequent row. Rod diameters are typically 0.5 mm or less to ensure low depositional loss of the aerosol.

The 3D rod arrays 903 and 1103 dissipate the turbulent air-jet leaving the inlet orifice 904 or 1104 from the air-jet DPI. The 3D rod arrays may also deaggregate the powder agglomerates in the aerosol. Dissipation of the air-jet prevents impaction of the aerosol in the patient interface and/or in the infant nostrils. Exemplary 3D rod arrays may be configured to allow aerosol penetration with less than 10% depositional loss of the aerosol by mass. It can be seen in the figures that exemplary 3D rod arrays need not extend wall-to-wall in a direction perpendicular to a long axis of the rods. The percentage of linear distance to the interface sidewall occupied by the rod array may be 5-50%, more preferentially, 10-30%, more preferentially approximately 15%. While more rods can be used, this minimized approach achieves the desired functional advantages while minimizing the cost associated with many rods. The distance from the capillary outlet to the nearest row of rods may be 0 to 5 mm, generally better at 1 to 2 mm, with an exemplary distance being 1.25 mm. Rod arrays may come in different dimensions for different embodiments. For the sake of non-limiting illustration, however, the following are some exemplary dimensional measures. Inlet capillary diameter may be 2.39 mm. Rod diameter may be 0.5 mm. All rods may have the same diameter or, in some cases, some rods may differ in diameter from other rods. Exemplary ranges in terms of capillary diameters are 1.25 mm to 7.5 mm.

The patient interface may comprise a smooth expansion of the sidewalls in the longitudinal direction of the patient interface, from at or near the inlet orifice to the end or past the longitudinal position at which the 3D rod array ends. The widening cross-section of the patient interface in the vicinity of the rods minimizes or avoids depositional loss on the sidewalls. Said differently, expanding the sidewalls in the vicinity of the 3D rod array and in the direction of jet dispersion maintains low deposition in the patient interface.

Figure 9, in addition to its inclusion of a 3D rod array immediately following the cannula 905 inlet orifice 904, comprises a flow division by bifurcation of cannula 905 into separated cannulas 906 and 907 which lead to bilateral infant nasal prongs 911. The position and arrangement of the rods in the 3D rod array is such that it contributes to the bifurcated flow into respective channels and minimizes depositional loss of aerosol by impaction at the initial point of bifurcation.

Figure 10A shows a patient interface 1000 a notable feature of which is a long gradual expansion region cannula 1010. Gradual expansion of the nasal cannula internal flow surfaces avoids flow separation and promotes laminar flow, which avoids aerosol depositional loss. The angle of the conical expansion on one side may be a 3-degree half angle. The length of the cannula 1010 may be for example 30 mm to 90 mm with a preferred range of 40-65 mm at a flow rate of 2-4 LPM. A pressure port 1011 is positioned near an end of the main cannula and near the start of the flexible prong 911. It is positioned as close as possible to the infant to best approximate lung pressure, but not interfere with delivery. A pressure sensor 1012 (shown via block diagram) is connected to the pressure port 1011. The pressure sensor 1012 may be incorporated into the patient interface itself or simply connected to it. The pressure sensor 1012 may be configured to monitor peak and average pressures that the infant lungs are exposed to and limit pressures to what is deemed safe by clinicians, e.g. 20-25 cm H₂O or in some cases as high as 45 cm H₂O. If a high value is detected, then the delivered air volume should be reduced until lung compliance is improved. Lung exposure pressure can be controlled or limited in several ways. First, a pressure limiting valve or pop-off valve common on manual ventilation bags may be included that opens if the pressure exceeds a specified safe amount, typically set to 35 cm H₂O in manual resuscitation. Secondly, if a delivered pressure is deemed too high by the physician, then the delivered air volume can be reduced using some of the specified gas sources before the next administered breath, which will reduce the exposed lung pressure as a function of the lung compliance.

Figure 10B is a variation on Figure 10A. The patient interface 1050 bears many of the same features as interface 1000 and is therefore similarly labeled. A chief difference however is the termination in a flexible nasopharyngeal tube 1051 instead of a nasal prong 911. The flexible nasopharyngeal tube 1051 advantageously avoids a need for full intubation of an infant while still bypassing the dead space of the nasal airways. Nasopharyngeal tube 1051 may be inserted through the nose and into the pharynx (throat) but ends upstream of the glottis. Length of a nasopharyngeal tube may be 3 to 6 cm depending on infant age.

Figure 11 has an expansion region 1107. In contrast with Figures 10A and 10B, the expansion region is a sudden, instantaneous expansion of the flow pathway at the transition between the capillary 1102 and interface cannula 1105, i.e. at opening 1104. In Figure 11 there is an open flow path above and below the rods. A distance after the rods the cannula gradually reduces in diameter in a region 1107 until to meets the size of the nasal prong 911.

Figure 12 is a patient interface 1200 comprising a jet diffuser 1203 which may be either low volume porous plate, as depicted by the left Section A-A inset, or a fine mesh, as depicted by the right Section A-A inset. In either case, the jet diffuser 1203 is placed just after the inlet orifice 1204. The cannula beginning at or before the inlet orifice 1204 and ending at or after the jet diffuser 1203 has a progressive enlarging cross-sectional size along the longitudinal direction of the cannula. The pores or mesh holes of the jet diffuser 1203 may be created by laser drilling or etching, for example. A pore size of 10-300 µm is suitable, with 100-200 µm being sometimes preferable, and 150 µm being exemplary for at least some embodiments.

Patient interfaces may be angled downward (according to the device orientation during use) at an angle of, for example 10-35 degrees, e.g. approx. 30 degrees, to allow the air-jet DPI system to be held level and still tightly seal to the patient nose. The angle of the patient interface with respect to a remainder of the air-jet DPI system may be achieved in the outlet capillary leaving the air-jet DPI component, an arrangement which generally produces no significant loss of powder. Example bends in air path are shown in Figures 2A/2B and Figures 6A-6F.

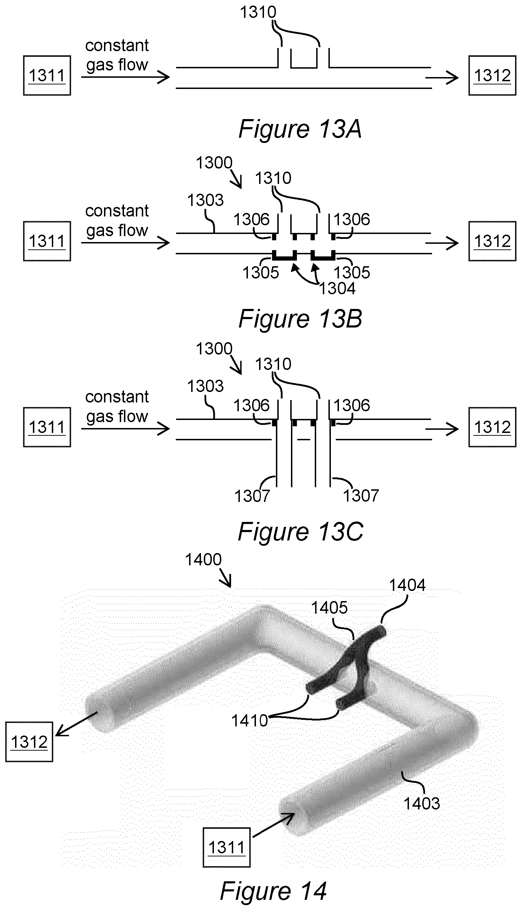

Figures 13A-13C concern embodiments having compatibility with continuous positive airway pressure (CPAP) setups. Nasal CPAP is a common form of ventilation support for infants. The system consists of a constant flow gas source, bilateral nasal prong interface, and downstream pressure regulation. Using this approach, the infant's respiration is supported with a constant additional air pressure of e.g. 5 cm H2O.

Figure 13A first shows a conventional CPAP setup. Constant gas flow from a gas source 1311 (e.g., O₂ blender) enters from the left and a pressure controller/regulator 1312, e.g. a water column, is arranged downstream to the right. The bilateral nasal prong interface 1310 is inserted into the infant's nostrils (e.g. approx. 10 mm) with the intent of forming an airtight seal with the nostril walls. Delivery of an aerosol to the gas stream of this conventional system results in a majority of the aerosol traveling past the infant with the excess gas flow required by nasal CPAP. Timing the aerosol bolus to reach the infant only during the brief ~0.3 s period of nasal inhalation is very difficult. Removing the infant from ventilation support for aerosol surfactant delivery may not be desirable.

Figures 13B and 13C show two configurations of a nasal CPAP rapid aerosol delivery system 1300 that overcomes the problems of the conventional system depicted by Figure 13A by connecting an air-jet DPI with an existing nasal CPAP bilateral nasal prong interface with an airtight seal with the infant's airways. No changes are necessary to the gas source 1311 or pressure regulator 1312. The tubing 1303 comprises nasal prongs which are configured to be inserted into the infant's nostrils and form an airtight seal with the nostrils. Unlike conventional CPAP systems, however, one or more access ports 1304 are positioned along the tubing 1303 near the nasal prongs and, in some exemplary embodiments, radially opposite the nasal prongs 1310 at the same longitudinal position of the tubing 1303 as the nasal prongs 1310. The access ports 1304 comprise removable/replaceable covers 1305 and may further include accessory receiving parts 1306. Partial flow openings remain between the covers 1305 and accessory receiving parts 1306 such that there is no significant effect on respiratory gas flow when the access ports 1304 are fully closed and therefore not in use.

Figure 13C shows the transition of the system 1300 to a second configuration used for administering an aerosol without removing the bilateral nasal prongs 1310 from the infant. The access port covers 1305 are removable to allow insertion of aerosol/surfactant delivery prongs 1307 (e.g., 911 of Figures 9-12) into the lumen of the tubing 1303. The prongs 1307 are advanced until they engage with the accessory receiving parts 1306 which may hold the prongs 1307 with a friction fit that is airtight. Fully connected with the friction fit, the delivery prongs 1307 are put in fluid communication with the nasal prongs 1310 and the infant's airways. In some embodiments the nasal prongs 1310 may be put in exclusive fluid communication with the delivery prongs 1307 such that air flow in the tubing 1303 is temporarily unable to reach the nasal prongs 1310. Alternatively, a nasopharyngeal tube may be inserted through the access port 1304, one nasal prong 1310, the nose, and into the pharynx for aerosol delivery. The other prong 1310 may be left open to the CPAP air or else is completely blocked. It should be appreciated that none of the original CPAP elements besides the covers 1305 need to be moved or otherwise changed. The openings created in the access ports 1304 by the removal of covers 1305 may be sized larger (e.g., in circumference or simply total cross-sectional area) than the delivery prongs 1307 such that one or more gaps exist between the delivery prongs 1307 and sides of the tubing 1303. The gaps allow excess CPAP air to exhaust into the environment during aerosol delivery. The delivery prongs 1307 may be left in place for, for example, a delivery period of approximately 3 breaths of the infant (at approx. 6 ml/kg). After the aerosol is delivered the delivery prongs 1307 are removed by gently pulling them away from the accessory receiving parts 1306, the access ports 1304 are closed once more by covers 1305, and traditional nasal CPAP is resumed.

Figure 14 shows another exemplary nasal CPAP rapid aerosol delivery system 1400, in particular a streamlined interface that enables high efficiency aerosol delivery from an air-jet or other positive pressure inline DPI during a period of infant inhalation. During typical nasal CPAP ventilation, the one access port 1404 is capped and the infant breaths freely through the bilateral nasal prongs 1410 (which are directly open to the ventilation gas flow in tubing 1403 from the gas source to the pressure controller 1312). When the aerosol is to be administered, the access port 1404 is opened and connected via an airtight flow passage to an air-jet (or other positive pressure) DPI, likely through a gradual expansion cannula (with or without a 3D rod array) such as those depicted in Figures 9-12. Upon infant inhalation, the air-jet DPI is actuated, which delivers the aerosol through the streamlined passage/conduit 1405 and into the bilateral nasal prongs 1410. The conduit 1405 may be configured to deliver aerosol to the one or more nasal prongs with no sudden changes in flow direction, e.g. with an S-curve shape. This configuration minimizes dead volume between the DPI device and infant lungs, increasing lung delivery of the aerosol. The conduit 1405 may have a total air volume of 0.5 ml or less. CPAP gas support remains continuous to apply positive pressure gas support to the lungs, even during exhalation.

### EXAMPLES

### Air-Jet DPI System

This Example provides data for a prototypical system 200 corresponding with Figures 2A and 2B and their accompanying descriptions above. Positive pressure gas source 201 was a hand-actuated syringe, and the patient interface 207 was a single-prong nasal interface to minimize changes to the aerosolization characteristics of the air-jet DPI 202. The other nostril was held closed in the experiments and the NT (nose throat) airway model was based on an infant with a closed oral airway.

Performance of the air-jet DPI approach was considered in two stages. In a first stage, aerosolization performance of the air-jet DPI through the end of the outlet flow passage (excluding the nasal interface) was evaluated for multiple internal flow pathway designs. To determine expected aerosolization performance for full-term and preterm infant conditions, aerosolization performance was assessed for AAVs of 30 ml and 10 ml, respectively. After evaluation of aerosolization performance, a second stage of assessment was conducted to determine the penetration of the aerosol through an infant NT *in vitro* model. Best performing devices from the first stage experiments were connected to a gradually expanding nasal interface which was then inserted (~5 mm) into a single nostril of a full-term infant NT model ending with a tracheal filter. For evaluation with the full-term NT model, air-jet DPIs were actuated with 30 ml of air. Aerosol deposition on the tracheal filter was taken as an approximation of lung delivery. In this Example, aerosol delivery through a preterm NT model was not assessed due to the limited amount of actuation air (10 ml) in comparison to the size of the tracheal filter housing (30 ml). Aerosol delivery with the full-term AAV of 30 ml is likely to penetrate through the filter housing and reach the filter substrate due to an airflow jet effect leaving the trachea.

It is noted that *in vitro* assessment of lung aerosol delivery frequently includes cyclic respiration of the model subject over an extended period of time. This aspect of the experimental setup is not realistic in evaluation of the air-jet DPI, because the subject's breath is delivered by the positive pressure actuation of the device, much as it would be when using a manual ventilation bag. The device is actuated rapidly, with actuation times <1 sec, and leaving the device in place for a brief period is used to facilitate a breath hold. Furthermore, because the device is operated with a manual syringe filled with air, exhalation into the aerosolization chamber of the device (which could degrade powder performance) does not occur.

The aerosolization chamber had the size zero capsule volume of 0.68 ml; however, the capsule was not included in the prototype as the device was pre-loaded. The inlet and outlet flow passage diameters had values of 0.6 mm and 0.9 mm, respectively.

The six air-jet configurations of Figures 6A to 6E were evaluated based on different inlet/outlet air flow pathways and aerosolization chamber geometries. Inlet and outlet conditions for each air-jet DPI of the six figures included the options of rounded (R), flush (F), protruding within the aerosolization chamber (P), and multiple inlets (M). The exemplary angle of 37° between the horizontal DPI and angle of patient interface where it meets the patient is based on the best fit after inserting the gradually expanding nasal interface into a flexible 3-D printed infant nose model and achieving an airtight seal.

The air-jet DPI designs of Figures 6A to 6E were constructed using 3D printed parts with a division through the aerosolization chamber to allow for loading powder into the device. After powder loading, the inlet and outlet components were sealed together using a twist-lock design and intermediate O-ring. In most cases, the inlet flow passage was created as part of the 3D printed model, except with designs D2 and D6 where a custom cut stainless steel (SAE 304) hollow capillary tube was used to form the protruding inlet. Inlet diameters were 0.6 mm in all cases except for D5, where three diverging inlets were used of diameters of 0.5 mm, and D6, where three inlets (d=0.5 mm) were again used but in a bundled configuration. All outlets included custom cut stainless steel capillaries with an internal diameter of 0.9 mm. Previous results have illustrated that very little spray dried powder is lost on the stainless steel surface under the operating conditions of the infant air-jet DPI. The 37° angle was formed by bending the capillary against a fixed curve for all designs except for D1, which includes the angle as part of the aerosolization chamber design. All parts, including the nasal interface were designed in SolidWorks (Dassault Systèmes, Paris, France), and exported as .STL files. The parts were then 3D printed at 32 µm resolution on a Stratasys Objet24 3D Printer (Stratasys Ltd., Eden Prairie, MN) using VeroWhitePlus resin. The parts were cleaned in a Stratasys waterjet cleaning station and allowed to dry before assembly.

### Nasal Interface Design

After initial aerosolization experiments, the lead air-jet DPIs were connected to an initial nasal interface design for aerosol delivery testing through the infant NT geometry. The nasal interface consisted of a straight gradually expanding circular cross-section. The design of the nasal interface with a length of 63 mm was based on computational fluid dynamics predictions that indicated a gradual expansion geometry could effectively slow the high-speed jet leaving the DPI with minimal aerosol loss and achieve the target outlet diameter of 4 mm for aerosol delivery to the infant nose. Dimensions of the nasal interface outlet section were based on a Hudson RCI Size No. 4 nasal CPAP cannula (Teleflex Medical, Research Triangle Park, NC) for a full-term infant. Resulting nasal interface inner and outer diameters at the outlet tip were 4 and 5.5 mm, respectively. A gradual exterior taper was included at the outlet of the nasal prong to help form an airtight seal with the infant's nostril. During aerosol delivery through the NT model, the single-prong nasal interface was inserted approximately 5 mm into one nostril and the other nostril was held closed.

### Full-Term Infant Nose-Throat (NT) Model

To test aerosol delivery efficiency to the lungs, administration was considered through a full-term NT airway model beginning at the nostrils and passing through the pharynx, larynx, and ending with a filter (Pulmoguard II, SDI Diagnostics, Easton, MA) at the start of the trachea. In this Example, the full-term NT model and delivery conditions were selected for a newborn infant with weight and height of 3550 g and 49.5 cm, respectively. Two methods that produced average age-appropriate depositions were achieved by scaling based on subject height or the *D*_{*V*/*As*} parameter. The *D*_{*V*/*As*} parameter is defined as the airway's volume divided by its surface area. However, since *D*_{*V*/*As*} is not known beyond the two points reported, scaling based on infant height is a reasonable approach. Average age-appropriate height data is readily available from growth charts and can be used for a scaling parameter. A number of studies have shown that subject height can be used as a parameter for scaling airway dimensions. A high-quality NT geometry of a 6-month-old infant that provided nasal airway deposition consistent with mean values was used. Based on infant body length (height) the appropriate geometric scaling factor to reduce this model to that of a new-born infant was 0.73. The resulting full-term newborn NT model employed in this Example had a volume of 3.6 ml and a nasal *D*_{*V*/*As*} of 0.94 mm.

The best characteristic length scale for collapsing impaction data to a single curve was *D*_{*V*/*As*} i.e., the *D*_{*V*/*As*} parameter is a good indicator for predicting age-appropriate nasal deposition. Since the full-term NT model employed in this Example has a *D*_{*V*/*As*} similar to other neonate models of the same age, the scaled NT model used is considerable a reasonable representative of full-term nasal conditions.

In order to provide a smooth and accurate internal airway surface, the middle passage and throat sections of the infant NT model were built using stereo-lithography (SLA) with Accura ClearVue resin through 3D Systems On Demand Manufacturing, resulting in a rigid model. To facilitate nasal interface prong insertion and the formation of an airtight seal, the anterior nose was constructed in flexible Agilus Translucent 30-A material, also using SLA from 3D Systems. During experimental testing, the separate regions were securely connected with a paraffin film lining and a small amount of lubrication on the interface surfaces to ensure an airtight seal.

### Evaluation of Flow Rate and Actuation

For each device tested, differences in air-jet design geometry led to different resistances that in turn alter the flow rate during actuation. Actuation was performed by hand after filling the 60 ml syringe (serving as the positive pressure gas source) to the desired AAV (either 10 ml or 30 ml) with room air and connecting to the device with a luer lock adapter. Quantification of the average flow rate for each device was performed using a pressure sensor (SSCDLNN040MBGSA5, Honeywell, Sensing and Control, Golden Valley, MN) affixed perpendicular to the outlet flow channel before the nasal interface. Pressure recordings (Sensor Evaluation Kit, Honeywell, Honeywell Sensing and Internet of Things, Fort Mill, SC) were taken at 500 samples/second. The pressure profile of the actuation was used to calculate average flow rate based on the fixed AAV and elapsed time. Elapsed time was determined by the number of samples with a pressure reading over a set threshold recoded during actuation, where the threshold was set to double the baseline pressure value.

### Evaluation of Air-Jet DPI Aerosolization Performance

The tests used 10 mg of AS-EEG powder formulation (manually weighed) and a Next Generation Impactor (NGI; MSP, TSI Incorporated, Shoreview, MN) for aerosol particle size analysis. After weighing, the powder mass was poured into the inlet half of the air-jet DPI, which was then assembled and sealed with a twisting motion. To assess the aerosol size distribution, the air-jet DPI (without the nasal interface) was attached to the pre-separator inlet of the NGI using a custom adapter. This adapter positioned the outlet of the air-jet DPI one cm away, perpendicular from the center of the pre-separator inlet with open space allowing for co-flow room air to enter the NGI, which was operated at a flow rate of 45 LPM using a downstream vacuum pump. Room temperature and relative humidity were recorded for every run and found to be between 21-24°C and 20-40%, respectively. The NGI was positioned 53° off horizontal to allow the device to remain level during use and maintain an inline flow path from the device outlet to the NGI inlet, as it would be during administration to a supine infant. Each stage of the NGI was coated with MOLYKOTE^{®} 316 silicone spray (Dow Corning, Midland, MI) to minimize particle bounce and re-entrainment. The NGI flow rate of 45 LPM was chosen to ensure collection of the aerosol, minimize any effects of settling, and provide appropriate stage cutoff diameters for evaluating small aerosol sizes. Before each set of experimental runs, the flow rate was confirmed using a flow sensor (Sensirion SFM3000, Sensirion AG, Stafa, Switzerland) connected to the NGI inlet.

Each device was actuated into the NGI via the 60 ml hand syringe at 30 ml or 10 ml AAV to compare aerosolization at full-term or preterm infant conditions. Three replicate runs for each device at each condition were performed in a randomized order. Analysis metrics included emitted dose (ED) and mass median aerodynamic diameter (MMAD). ED was calculated as the mass of AS in the loaded dose minus the mass of AS remaining in the device divided by the initial loaded mass of AS. Aerosolization calculations were based on the mass of AS recovered in the NGI. MMAD/ED was also used as a general parameter to indicate overall performance (lower values being preferable). Drug masses were determined using HPLC analysis, as described below.

### Evaluation of Lung Delivery in the Full-Term NT Model

Based on device assessment, the second stage of this Example used the three best performing designs for full-term NT *in vitro* model testing at an AAV of 30 ml. The experimental setup was the same as for device assessment in terms of device actuation, powder loading, and randomization. However, instead of the device connecting to the NGI adapter, it was connected to the gradually expanding nasal interface which was inserted approximately 5 mm into the left nostril of the infant NT model (the right nostril was manually held closed during actuation). A small amount of lubrication was applied to the exterior of the prong to ensure and airtight seal. All NT model segments were internally coated with silicon spray to minimize particle bounce similar to airway surface liquid. At the end of the NT model, a respiratory filter (Pulmoguard II, SDI Diagnostics, Easton, MA) collected powder passing through the extrathoracic regions and represented the amount of drug delivered to the lung. After aerosol delivery, the nose was held closed and the syringe was not disconnected for 10 seconds to help aid in the capture of all particles. In practice, a breath-hold such as this would help prevent drug loss during expiration as the EEG particles significantly increase in size through hygroscopic growth. Calculations for ED and regional deposition, including the nasal interface and in the NT model and tracheal filter (amount deliver to lung) were expressed as a percentage of the loaded dose of AS.

### Drug Mass Characterization Methods

After actuation and aerosolization, drug masses retained or collected in the air-jet DPI and NGI or nasal interface, NT model, and filter were recovered by dissolving in an appropriate volume of deionized water followed by high performance liquid chromatography (HPLC) analysis. The loaded drug mass was determined through content uniformity analysis of the AS-EEG formulation; where known masses of AS-EEG were dissolved in water and the AS content (µg/mg of formulation) was determined. AS quantification was performed for each deposition site and for the drug mass used to calculate the drug recovery. Drug recovery percentage was expressed as the amount of AS recovered on all deposition sites divided by the loaded AS dose for each experiment.

Based on an airflow rate of 45 LPM, the NGI stage cut-off diameters were determined using the formula specified in United States Pharmacopeia (USP 35) (Chapter 601, Apparatus 5). The MMAD was calculated through linear interpolation between appropriate stages using a plot of cumulative percentage drug mass vs. cut-off diameter.

### Powder Formulation Characterization Method

In preliminary experiments prior to device testing with the NGI and infant NT model, primary particle size of the AS-EEG powder formulation was determined by laser diffraction using the Sympatec HELOS (submicron R1 lens with 20 mm focal length) with RODOS/M disperser at 4 bar, and ASPIROS sample feeder set to 60 mm/sec (Sympatec GmbH, Clausthal-Zellerfeld, Germany). Three consecutive samples were tested on the same day. Testing at the high pressure of 4 bar is intended to show maximum particle dispersion. This primary particle size then serves as a benchmark to evaluate the aerosolization efficiency of the air-jet DPI, which is operated with 1000x - 10,000x less pressure.

### Statistical Analysis

Statistical analysis for comparing aerosolization performance across all devices and comparison of device performance at different AAVs was performed using JMP Pro 15 (SAS Institute Inc., Cary, NC). Comparison of device performance utilized one-way ANOVA followed by post hoc Tukey. Comparison of preterm vs. full-term AAVs for each device were performed with the Students t-test. All statistical tests used a significance limit of P=0.05.

### Results

### Powder and Air Flow Rate

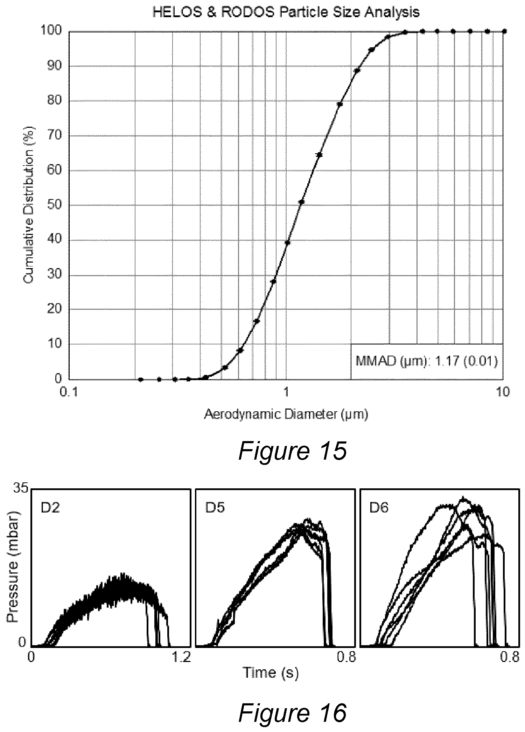

The AS-EEG formulation was characterized as having a mean (SD) geometric diameter of 0.99 (0.0) µm. In order to compare laser diffraction primary particle size to the air-jet DPI performance, the geometric size (measured with laser diffraction) was converted to an aerodynamic diameter using a theoretical solid particle density of 1.393 g/cm³ (based on weighted particle densities of the formulation components). Figure 15 shows the mean cumulative aerodynamic diameter size distribution of the AS-EEG powder formulation, resulting in a mean MMAD of 1.17 µm. This size is expected to be near full dispersion based on the high pressure used with the laboratory scale dispersion unit.

Time-averaged air flow rates were determined for each device actuated by hand six times, with the mean (SD) results reported in Table 2 for an AAV of 30 ml. Figure 16 shows the pressure profile during actuation for D2, D5, and D6, which characterize pressure profiles for all devices tested. A fixed volume of 30 ml and the average total time for each device were used to calculate the time-averaged flow rate. D2 was found to have the lowest time-averaged flow rate of 1.9 LPM while D5 and D6 had the highest of 2.7 LPM. In the same manner, the time-average flow rates for each device were calculated with an AAV of 10 ml, with the results provided in Table 3. There was no statistically significant change in average flow rate between the two AAVs, with the exception of D6, which was slightly, but significantly lower at 10 ml (t-test, P=0.007). All devices have acceptable time-averaged flow rates within the target 1-5 LPM for infant aerosol delivery.

**Table 2: Aerosolization performance and measured flow rate with an actuation air-volume (AAV) of 30 ml.**

| **DPI Design^{a}** | **Flow Rate (LPM)¹** | **ED (%)²** | **MMAD (µm)²** | **MMAD/ED²** |
|---|---|---|---|---|
| D1 | 2.0 (0.1) | 88.1 (0.5) | 1.88 (0.10) | 0.0214 (0.0012) |
| D2 | 1.9 (0.1) | 75.6 (1.5) | 1.58 (0.03) | 0.0210 (0.0007) |
| D3 | 2.0 (0.1) | 79.4 (0.9) | 1.71 (0.02) | 0.0216 (0.0005) |
| D4 | 2.2 (0.1) | 69.2 (1.1) | 1.58 (0.05) | 0.0228 (0.0010) |
| D5 | 2.7 (0.1) | 94.1 (0.6) | 1.86 (0.02) | 0.0198 (0.0003) |
| D6 | 2.7 (0.2) | 85.9 (1.1) | 1.71 (0.04) | 0.0200 (0.0003) |
| Mean values with standard deviations (SD) shown in parenthesis; ¹n=6, ²n=3. | | | | |
| ^{a}*p*<0.05 significant effect of device design on each of the individual reported parameters (one-way ANOVA). | | | | |
| LPM, liters per minute (L/min); ED, emitted dose; MMAD, mass median aerodynamic diameter. | | | | |

### Performance of Air-Jet DPIs

Aerosolization metrics in terms of ED and MMAD for full-term and preterm AAVs of 30 ml and 10 ml are presented in Tables 2 and 3, respectively. Surprisingly, all devices tested produced an aerosol MMAD below 2 µm at both AAVs. ED values were also relatively high with multiple devices emptying over 85% of the loaded dose. The highest ED values were from designs D5 (94.1%) and D1 (88.1%) actuated with 30 ml of air. Choosing best device performance based only on aerosolization metrics is difficult as increasing ED is often associated with increasing MMAD, which may have a net negative effect on aerosol delivery to the trachea and lungs. A potentially useful metric is MMAD/ED, where lower values are desirable and expected to improve aerosol delivery to the lungs. For both AAVs, the lowest three values of MMAD/ED were provided by designs D5, D6, and D2 (using full-term AAV conditions the MMAD/ED values were 0.0198, 0.0200, and 0.0210, respectively). Examining Table 2 for the 30 ml AAV, it was observed that there were statistically significant differences among the different device designs for each aerosolization performance parameters. For the MMAD/ED metric, D4 design with the highest value MMAD/ED was observed to be significantly different to both designs D5 and D6, with the two lowest MMAD/ED values (post hoc Tukey: P=0.003 and 0.006, respectively).

**Table 3: Aerosolization performance and measured flow rate with an AAV of 10 ml.**

| **DPI Design^{a}** | **Flow Rate (LPM)¹** | **ED (%)²** | **MMAD (µm)²** | **MMAD/ED²** |
|---|---|---|---|---|
| D1 | 2.1 (0.1) | 86.8 (0.2) | 1.95 (0.06) | 0.0224 (0.0007) |
| D2 | 2.0 (0.1) | 72.5 (1.5) | 1.55 (0.04) | 0.0214 (0.0002) |
| D3 | 2.0 (0.1) | 73.0 (2.2) | 1.65 (0.03) | 0.0226 (0.0007) |
| D4 | 2.2 (0.1) | 67.9 (0.6) | 1.64 (0.05) | 0.0241 (0.0007) |
| D5 | 2.6 (0.0) | 92.7 (0.6) | 1.85 (0.06) | 0.0199 (0.0008) |
| D6 | 2.3 (0.1) | 82.7 (0.9) | 1.66 (0.03) | 0.0201 (0.0005) |
| Mean values with standard deviations (SD) shown in parenthesis; ¹n=6, ²n=3. | | | | |
| ^{a}*p*<0.05 significant effect of device design on each of the individual reported parameters (one-way ANOVA). | | | | |

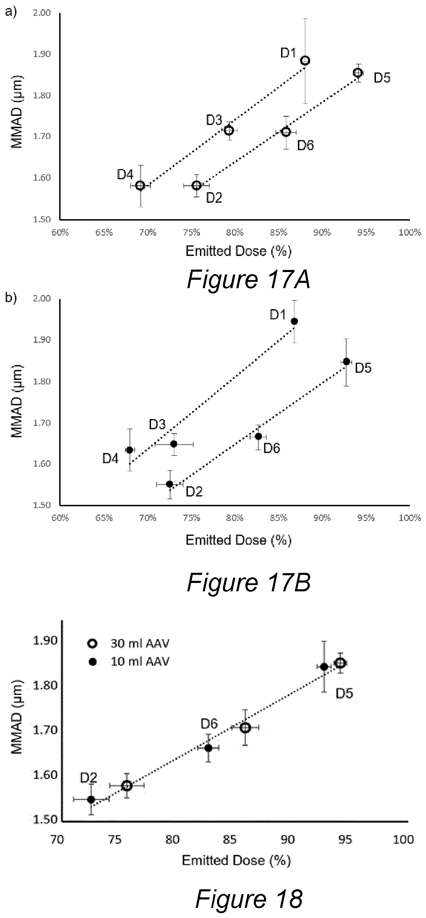

To assess overall DPI performance, it is useful to graph MMAD vs. ED, as shown in Figure 17A and 17B for the two AAVs considered (30 ml and 10 ml, respectively). In these figures, at each AAV, two distinct sets of DPI performance are observed, which fall along linear best-fit lines of MMAD vs. ED. If the linear performance curve formed by D1, D3, and D4 is considered as a reference, then improved performance is achieved by devices that fall in the space below and to the right of this line. As a result, devices D2, D5, and D6 are shown to have overall improved performance compared with D1, D3, and D4. Viewed another way, if an ED of 85% is set as a performance target, both D1 and D6 are acceptable, but D6 reduces the MMAD at the same ED by approximately 0.1-0.3 µm. Fortunately, the group of D2, D5, and D6 perform better than the other designs at both AAVs. Comparing performance between AAVs of 30 ml and 10 ml, D5 shows an increase in MMAD variability, which is not present with D2 or D6.

Figure 18 provides a comparison of the three best performing (based on ranking of MMAD/ED and linear best-fit lines seen in Figures 17A and 17B) DPI designs D2, D5, and D6 actuated at 30 ml and 10 ml. It is observed that reducing the AAV by a factor of 3-fold has little influence on device performance, based on similarity in the data points for each device along the best-fit curve. Table 4 reorganizes the data from Tables 2 and 3 to show a side-by-side comparison of the best performing devices operated at each AAV. Statistical analysis (t-test) was performed for each device comparing performance at the two AAVs. While it can be seen that the lower AAV (10 ml) corresponds with a slightly but statistically significant lower ED, there was no statistical difference for MMAD or the MMAD/ED parameter. Based on results from Table 4 and similarity in the data plot locations (Figure 18), it is observed that device D5 was least influenced by the 3-fold reduction in AAV.

**Table 4: Comparison of best design aerosolization performance at 10 and 30 ml AAVs.**

| **Description** | | **10 ml AAV** | **30 ml AAV** |
|---|---|---|---|
| **D2** | | | |
| | ED (%)^{a} | 72.5 (1.5) | 75.6(1.5) |
| | MMAD (µm) | 1.55 (0.04) | 1.58 (0.03) |
| | MMAD/ED | 2.14 (0.02) | 2.10 (0.07) |

| **D5** | | | |
|---|---|---|---|
| | ED (%)^{a} | 92.7 (0.6) | 94.1 (0.6) |
| | MMAD (µm) | 1.85 (0.06) | 1.86 (0.02) |
| | MMAD/ED | 1.99 (0.08) | 1.98 (0.03) |

| **D6** | | | |
|---|---|---|---|
| | ED (%)^{a} | 82.7 (0.9) | 85.9(1.1) |
| | MMAD (µm) | 1.66 (0.03) | 1.71 (0.04) |
| | MMAD/ED | 2.01 (0.05) | 2.00 (0.03) |
| Mean values with standard deviations (SD) shown in parenthesis, n=3. | | | |
| ^{a}*p*<0.05 significant difference between 10 & 30 ml AAV (Student's t-test) | | | |

### Nose-to-Lung (N2L) Aerosol Delivery

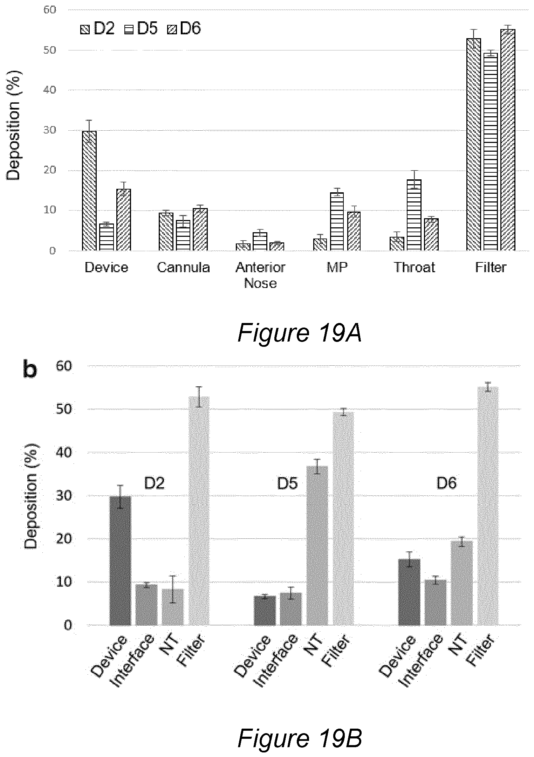

Best performing devices D2, D5, and D6 were connected to the gradually expanding nasal interface and tested for aerosol delivery through the full-term NT model at an AAV of 30 ml. Deposition fractions in each region of the delivery system and NT model are displayed in Figures 19A and 19B. Considering drug mass retained in the device, connecting to the nasal interface and NT model moderately increased retention in device D2 to about 29%, while the drug retention for devices D5 and D6 slightly decreased to about 5% and 15%, respectively. All delivery systems lost approximately 7-10% in the gradually expanding nasal interface, which is significantly better than 30-40% loss in preliminary rapidly expanding nasal interfaces. In the nose, highest deposition was seen in the middle passage (MP) and throat regions.

Surprisingly, results showed that depositional losses in different regions tend to provide similar total values leading to a narrow range of 48-53% lung delivery efficiency. Reasons for similar lung delivery efficiencies across the three devices tested can be further explored in Figure 19B and Table 5. These results present aerosol deposition (or retention) fractions grouped for each device and subdivided for the nasal interface, complete NT regions, and tracheal filter (lung delivery). Despite statistically significant differences between designs in all deposition regions leading up to the tracheal filter, there was no significant difference in filter deposition (lung delivery). Interestingly, it is observed that the D2 device generating the smallest aerosol (1.55 µm), produced only 8.0% NT depositional loss, while increasing aerosol size to 1.85 µm with D5 dramatically increased nasal depositional loss to 36.1%. With D2, the low nasal depositional loss is offset by approximately 29% device retention, resulting in a tracheal filter deposition fraction of 52.2%. Device D5 reduces DPI retention to about only 5%, but then dramatically increased NT deposition leading to a similar 48.5% lung delivery efficiency. Average total AS recovery percentages for the best performing designs were between 95-98% ensuring mass balance and validity of the experiments.

**Table 5: Regional deposition fractions (based on loaded dose) for air-jet DPI designs in full-term nose-throat (NT) model and 30 ml AAV.**

| **Deposition Region** | **D2** | **D5** | **D6** |
|---|---|---|---|
| DPI Retention (%)^{a} | 29.4 (3.4) | 4.5 (0.4) | 14.5 (1.7) |
| Cannula (%)^{a} | 9.2 (0.5) | 7.3 (1.2) | 10.0 (0.9) |
| Anterior Nose (%)^{a} | 1.7 (0.7) | 4.5 (0.7) | 1.9 (0.4) |
| Middle Passage (%)^{a} | 2.9 (1.1) | 14.3 (0.9) | 9.1 (1.3) |
| Throat (%)^{a} | 3.5 (1.0) | 17.4(2.5) | 7.4 (0.7) |
| Total Extrathoracic (%)^{a} | 8.0 (2.9) | 36.1 (2.5) | 18.4 (1.0) |
| Tracheal Filter (%) | 52.2 (2.8) | 48.5 (1.4) | 52.6 (1.2) |
| Mean values with standard deviations (SD) shown in parenthesis, n=3. | | | |
| Total Extrathoracic is the sum of anterior nose, middle passage and throat deposition. | | | |
| ^{a}*p*<0.05 significant effect of device design on deposition (one-way ANOVA). | | | |

### Discussion

The new infant air-jet DPI prototypes tested in this Example achieved performance metrics of>80% ED, MMAD <1.8 µm, and a lung delivery efficiency of approximately 50% of device loaded dose. In assessing the air-jet DPI designs, two distinct sets of devices were identified based on aerosolization performance. Designs D2, D5, and D6 were found to produce a superior combination of MMAD and ED (based on MMAD/ED and linear best-fit lines) than the remaining three devices. Common design features of the three best performing air-jet designs were a cylindrical and horizontal aerosolization chamber together with a flush or protruding outlet. Designs that also included multiple inlets (D5 and D6) achieved the best aerosolization metrics of approximately >80% ED and MMAD <1.8µm, whereas D2 with a single air inlet did not. Nevertheless, when tested in conjunction with a gradually expanding nasal interface design and infant NT model, all three lead designs achieved approximately 50% drug delivery to the lungs. Furthermore, it is expected that the lung delivery efficiency can be increased beyond 50% by improved regulation of the input flow profile and by inclusion of a 3D rod array in the nasal interface to better dissipate the turbulent jet and further deaggregate the aerosol entering the nose, thereby further reducing the aerosol MMAD and reducing the nasal deposition fraction.

The D5 design appears to perform better than previous devices with an ED of 94% and small aerosol size increase to 1.85 µm. As a result, this Example indicates that air-jet performance was enhanced through the development of an aerosolization chamber with multiple inlets and a flush or protruding outlet.

Despite significant variation in the best performing devices (D2, D5, and D6 with an ED range of 75.6-94.1%), *in vitro* lung delivery efficiency was consistently around 50% using the full-term NT geometry. As described, the D2 design provided excellent NT penetration with only 8.0% depositional loss, but relatively poor emptying with ED of 75.6% (71.6% when connected to the infant NT model). Increasing the device emptying also increased the MMAD, which led to a significant depositional loss in the NT model. An exemplary device for infant N2L aerosol delivery therefore appears to be one that can achieve an MMAD of 1.6 µm and ED near 95%.

Intended uses of the infant air-jet DPI include the rapid administration of high dose inhaled medications such as aerosolized antibiotics and surfactants. For either of these applications, total doses higher than the 10 mg loaded dose employed in this study will likely be needed. The aerosolization chamber in the current study has a total volume of 0.68 ml, which if approximately half full can accommodate 40-50 mg of powder based on typical EEG powder density. An alternative to accommodate higher total dose loading may be an auto-loading system for the air-jet DPI such that each actuation delivers a 10 mg dose of powder formulation. Figures 7A, 7B, 8A, and 8B and their corresponding descriptions above show and describe some exemplary auto-loading systems.

As demonstrated in this Example, the infant air-jet DPI is expected to provide a number of advantages for aerosol delivery to infants of all ages. First, device actuations of 10 ml and 30 ml require time periods between 0.2 and 1.0 s. The devices tested performed very similar at both AAVs in terms of aerosolization performance. Therefore, the only reason to implement the higher AAV for the older full-term infant is to provide a full inhalation breath. This air volume can be reduced to accommodate stiff or non-compliant lungs as needed. By operating the delivery system with positive pressure, it is expected that the highly flexible infant upper airways will be expanded, enabling better deep lung penetration of the aerosol. In the current Example an enforced breath hold of 10 s was implemented; however, this length of time is not needed for aerosol retention as EEG aerosols approach their fully hydrated droplet size within approximately 0.5 to 1 s under infant airway conditions. Nevertheless, under resuscitation conditions, infant lung inflation followed by a 10 s breath hold improves lung mechanics and patient outcomes compared with standard rapid ventilation with a bag and mask. With a single-prong design, infant exhalation can be accomplished by releasing the nostril without the nasal interface. For a dual-prong design, an exhalation port is included in the nasal interface close to the patient. Opening of the exhalation port can be automated with a single button on the device that also controls actuation of the air source.

The *in vitro* NT model employed in the current Example has several differences from *in vivo* conditions that should be considered. As described previously, the air-jet DPI delivers both the aerosol and a full inhalation breath such that cyclic breathing of the model is not required. However, more realistic airway delivery conditions need to include the downstream resistance and compliance of the lungs. The effect of this resistance and compliance on aerosol generation is expected to be small considering the relatively low ventilation volumes (7-8 ml/kg) that are employed. Furthermore, the airway walls were not warmed and humidified to physiologic conditions. It is known that some size increase of hygroscopic aerosols occurs in the nose. However, this aerosol size increase in the extrathoracic region is small with an associated negligible increase in NT deposition (<5% relative difference) for adult airway conditions. The air-jet DPI forms a closed system with the airways such that subject exhalation into the aerosolization chamber containing the powder is not possible.

Variations in airflow delivery associated with hand-operation of the gas source are observed in Figure 16. Despite these variations, performance of the air-jet DPI was relatively consistent with acceptable standard deviation values.

The nasal interface is an important source of potential aerosol loss and also influences loss in the NT model. The single gradual expansion nasal interface used in this study was the result of preliminary design work that improved upon nasal interface losses as high as 40% of the loaded dose. While 10% nasal interface depositional loss is acceptable, it is expected that this deposition can be further reduced, likely through optimization of the inlet flow profile and/or inclusion of a 3D rod array structure in the interface designed to disperse the turbulent jet and further deaggregate the powder.

Finally, this Example considered only one NT model under full-term neonate conditions. Aerosol deposition in the NT region is known to be highly variable and where the tested model falls within this spectrum is currently not known.

In conclusion, this Example tests a prototype air-jet aerosol delivery system to administer high doses of spray-dried powder formulations to infants. The patient interface is a simple gradually expanding flow passage that produced low depositional loss and device actuation times are in a range between 0.2 and 1.0 s. Delivery efficiency of drug to the lungs was approximately 50% of the loaded dose across the three best performing devices. Advantageous design options in the air-jet DPI were identified as a horizontal and cylindrical aerosolization chamber, flush or protruding outlet, and multiple inlets.

Intended applications of exemplary infant air-jet DPIs and DPI systems are the delivery of higher dose inhaled medications where efficacy can be increased with improved lung and deep lung targeting, and where reduced inter- and intra-subject variability is important. Potential candidate medications include inhaled antibiotics, growth hormone, anti-virals, gene therapies for lung diseases, bronchodilators and corticosteroids for asthma management, surfactants, clearance agents, insulin, and anti-inflammatories.

It is to be understood that the terminology used herein is for the purpose of describing particular embodiments only, and is not intended to be limiting, since the scope of the present invention will be limited only by the appended claims.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be separated from or combined with the features of any of the other several embodiments without departing from the scope or spirit of the present invention. Any recited method can be carried out in the order of events recited or in any other order which is logically possible.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are described.

### Embodiments

1. An air jet dry powder inhaler (DPI) system for infants, comprising
   a gas source configured to deliver in a single actuation up to but not exceeding a full inhalation breath volume for an infant;
   a patient interface configured to form an airtight seal with one or both of an infant's nostrils; and
   a fixed position air jet DPI arranged inline between the gas source and the patient interface and configured to introduce an aerosol to gas from the gas source before the gas reaches the patient interface,
   wherein the air jet DPI system is configured to have airtight communication with lungs of the infant when the patient interface is forming an airtight seal with one or both of the infant's nostrils and all other pulmonary orifices are closed,
   wherein the air jet DPI system delivers both the aerosol and the full inhalation breath volume for an infant using positive-pressure gas.
2. The DPI system of embodiment 1, wherein the full inhalation breath volume is a maximum of 100 ml or less.
3. The DPI system of embodiment 1, wherein the full inhalation breath volume is a maximum of 10 ml or less.
4. The DPI system of embodiment 1, wherein a total air space volume of the air jet DPI system is 5ml or less.
5. The DPI system of embodiment 4, wherein a total air space volume of the air jet DPI system is 2 ml or less.
6. The DPI system of embodiment 1, wherein the gas source is configured to deliver full inhalation breath volume for an infant to the patient through the patient interface in one second or less.
7. The DPI system of embodiment 1, wherein the gas source comprises one or more hand actuated syringes.
8. The DPI system of embodiment 7, wherein the one or more hand actuated syringes comprise one or more springs.
9. The DPI system of embodiment 1, wherein the gas source is a compressed gas source.
10. The DPI system of embodiment 9, wherein the gas source is a bank of gas syringes compressible prior to actuation.
11. The DPI system of embodiment 10, wherein the gas syringes are individually actuatable.
12. The DPI system of embodiment 1, wherein a cannula of the patient interface has a gradually expanding interior over a length of 40-80 mm.
13. The DPI system of embodiment 1, wherein the patient interface comprises a nasopharyngeal tube.
14. The DPI system of embodiment 1, further comprising a bend in flow path downstream of the air jet DPI of 10° to 45°.
15. The air jet DPI system of embodiment 1, further comprising
   a drying chamber; and
   a one-way valve configured for admitting air from the environment into the air jet DPI system when the gas source volume is expanding.
16. The DPI system of embodiment 15, wherein the drying chamber houses a powder free desiccant.
17. The DPI system of embodiment 1, wherein the air jet DPI comprises
   an elongate aerosolization chamber with a longitudinal axis, and
   one or more inlets and one or more outlets all positioned at an upper longitudinal segment of the aerosolization chamber,
   wherein the upper longitudinal segment extends no more than 50% of a length of the aerosolization chamber.
18. The DPI system of embodiment 17, wherein the upper longitudinal segment extends no more than 25% of the length of the aerosolization chamber.
19. The DPI system of embodiment 1, wherein the patient interface comprises a 3D rod array arranged such that an aerosol jet entering the patient interface must pass through the 3D rod array before exiting the patient interface.
20. The DPI system of embodiment 19, wherein the 3D rod array comprises a plurality of rows of rods which extend between opposite walls of a lumen of the patient interface.
21. The DPI system of embodiment 20, wherein the 3D rod array spans less than an entire cross-sectional distance of the lumen between the at least one inlet and the one or more exit orifices in a direction perpendicular to a long axis of the rods of the 3D rod array.
22. The DPI system of embodiment 21, wherein the 3D rod array is spaced 0 to 5 mm away from the at least one inlet orifice along a primary flow axis of the lumen.
23. The DPI system of embodiment 22, wherein the 3D rod array is spaced 1 to 2 mm away from the at least one inlet along the primary flow axis of the lumen.
24. A gas source for an air jet dry powder inhaler (DPI) usable with infants, comprising
   a barrel sized to retain 100 ml or less of gas, the barrel having an exit orifice at one end;
   a rod;
   a piston and gasket sealing an end of the barrel opposite the exit orifice and moveable by the rod to change an internal volume of the barrel by driving gas into or out of the exit orifice;
   a handle piece configured such that the rod, piston, and gasket are moveable a maximum displacement to deliver a predetermined volume of air through the exit orifice using a single squeeze.
25. A multidose storage and delivery unit (MDU) for dosing dry powder into an air jet dry powder inhaler (DPI), comprising
   a main body sized to accommodate 100 mg or less of powder and configured to attach in an airtight manner to an aerosolization chamber of the air jet DPI;
   a release mechanism for releasing predetermined doses from the main body into the aerosolization chamber of the air jet DPI upon satisfaction of a predetermined condition.
26. The MDU of embodiment 25, wherein the main body is divided into compartments, each compartment accommodating a separate dose of the powder.
27. The MDU of embodiment 26, wherein the release mechanism is a rotatable retaining disk, and wherein the predetermined condition is a rotation of the retaining disk.
28. The MDU of embodiment 25, wherein the release mechanism is a mesh or plate containing one or more small openings sized to limit or prevent passage of powder solely under the force of gravity, wherein the predetermined condition is a change in air flow or pressure at the one or more small openings.
29. An aerosol delivery system for infants, comprising
   a tubing for transporting a continuous positive airway pressure (CPAP) air supply;
   nasal prongs sealable with an infant's nostrils;
   at least one access port along the tubing next to the nasal prongs, the at least one access port comprising a temporarily removable cover and a receiving part, wherein the at least one access port is sized to allow passage of an aerosol delivery prong into a lumen of the tubing, and wherein the receiving part is sized to form an airtight seal with an end of the aerosol delivery prong which puts the delivery prong in fluid communication with at least one of the nasal prongs and the infant's airways.
30. An aerosol delivery system for infants, comprising
   a tubing for transporting a continuous positive airway pressure (CPAP) air supply; and
   one or more nasal prongs sealable with an infant's nostrils, wherein the one or more nasal prongs are in fluid communication with a lumen of the tubing and a conduit connecting with at least one access port independent of the tubing to which an aerosol delivery prong is connectable.
31. The aerosol delivery system of embodiment 30, wherein the conduit between the access port and the one or more nasal prongs allows the one or more nasal prongs to remain open to CPAP air supply.
32. The aerosol delivery system of embodiment 30, wherein the conduit between the access port and the one or more nasal prongs is configured to deliver aerosol to the one or more nasal prongs with no sudden changes in flow direction.
33. The aerosol delivery system of embodiment 32, wherein the conduit has an S-curve shape.
34. The aerosol delivery system of embodiment 30, wherein the conduit has a total air volume of 0.5 ml or less.

## Claims

1. An aerosol delivery system (1300) for infants, comprising
a tubing (1303) for transporting a continuous positive airway pressure (CPAP) air supply;
nasal prongs (1310) sealable with an infant's nostrils;
at least one access port (1304) along the tubing (1303) next to the nasal prongs (1310), the at least one access port (1304) comprising a temporarily removable cover (1305) and a receiving part (1306), wherein the at least one access port (1304) is sized to allow passage of an aerosol delivery prong (1307) into a lumen of the tubing, and wherein the receiving part (1306) is sized to form an airtight seal with an end of the aerosol delivery prong (1307) which puts the delivery prong (1307) in fluid communication with at least one of the nasal prongs (1310) and the infant's airways.

2. An aerosol delivery system (1400) for infants, comprising
a tubing (1403) for transporting a continuous positive airway pressure (CPAP) air supply; and
one or more nasal prongs (1410) sealable with an infant's nostrils, wherein the one or more nasal prongs (1410) are in fluid communication with a lumen of the tubing (1403) and a conduit (1405) connecting with at least one access port (1404) independent of the tubing (1403) to which an aerosol delivery prong is connectable.

3. The aerosol delivery system (1400) for infants according to claim 2, wherein the conduit (1405) between the access port (1404) and the one or more nasal prongs (1410) is configured to deliver aerosol to the one or more nasal prongs (1410) with no sudden changes in flow direction.

4. The aerosol delivery system (1400) for infants according to any one of claims 2-3, wherein the conduit (1405) between the access port (1404) and the one or more nasal prongs allows the one or more nasal prongs (1410) to remain open to CPAP air supply.

5. The aerosol delivery system (1400) for infants according to any one of claims 2-4, wherein the conduit (1405) has an S-curve shape.

6. The aerosol delivery system (1400) for infants according to any one of claims 2-5, wherein the conduit (1405) has a total air volume of 0.5 ml or less.
